# EUROPEAN PATENT SPECIFICATION

(11) **EP 2 843 049 B1**
(45) Date of publication and mention of the grant of the patent: **11.04.2018**
(21) Application number: 13781077.6
(22) Date of filing: 26.04.2013
(51) Int. Cl.: A61K 31/4439, A61K 31/7088, A61K 31/7105, A61K 31/713, A61K 35/30, A61K 35/76, A61K 45/00, A61K 48/00, A61P 25/00, A61P 25/14, A61P 25/28, A61P 43/00, C12N 5/0793, C12N 5/0797, C12N 15/09, C12N 5/10, C12N 15/113

(54) **NEURONAL DIFFERENTIATION PROMOTER**
PROMOTER FÜR NEURONALE DIFFERENZIERUNG
PROMOTEUR DE DIFFÉRENCIATION NEURONALE

(30) Priority: 27.04.2012 JP 2012103875
(43) Date of publication of application: 04.03.2015
(73) Proprietor: Keio University, Tokyo 108-8345 (JP)
(72) Inventor: KANEDA, Hayato, Yokohama-shi Kanagawa 230-0045 (JP); OKANO, Hideyuki, Tokyo 160-8582 (JP); SHIMAZAKI, Takuya, Tokyo 160-8582 (JP)
(74) Representative: Gill Jennings & Every LLP
(86) International application number: PCT/JP2013/062490
(87) International publication number: WO 2013/162027

(56) References cited:
- WO-A1-00/17175
- WO-A1-01/01986
- WO-A1-2007/030870
- WO-A1-2010/018652
- WO-A1-2012/154814
- WO-A2-00/12497
- WO-A2-2005/039393
- US-A1- 2012 245 188
- NEW L ET AL.: 'SB203580 Promote EGF-Stimulated Early Morphological Differentiation in PC12 Cell Through Activating ERK Pathway' J CELL BIOCHEM vol. 83, no. 4, 2001, pages 585 - 596, XP055172901
- GU, P ET AL.: 'p38 signal-transduction pathway mediates the differentiation of mesencephalic neural stem cells induced by bone marrow stromal cell-conditioned medium' JOURNAL OF CLINICAL REHABILITATIVE TISSUE ENGINEERING RESEARCH vol. 11, no. 37, 2007, pages 7329 - 7332, XP008175283
- HORIUCHI H ET AL.: 'Continuous intrathecal infusion of SB203580, a selective inhibitor of p38 mitogen-activated protein kinase, zeduces the damage of hind-limb function after thoracic spinal cord injury in rat' NEUROSCI RES. vol. 47, no. 2, 2003, pages 209 - 217, XP002460466
- FAIGLE R ET AL.: 'ASK1 inhibits astroglial development via p38 mitogen-activated protein kinase and promotes neuronal differentiation in adult hippocampus-derived progenitor cells' MOL CELL BIOL. vol. 24, no. 1, 2004, pages 280 - 293, XP055172922
- TROMPETER HI ET AL.: 'MicroRNAs MiR-17, MiR- 20a, and MiR-106b act in concert to modulate E2F activity on cell cycle arrest during neuronal lineage differentiation of USSC' PLOS ONE. vol. 6, no. 1, 2011, page E16138, XP055172924
- SATO K ET AL.: 'Inhibitors of p38 mitogen- activated protein kinase enhance proliferation of mouse neural stem cells' J NEUROSCI RES. vol. 86, no. 10, 2008, pages 2179 - 2189, XP055172929
- YANG S-R ET AL.: 'Role of Gap Junctional Intercellular Communication (GJIC) through p38 and ERK1/2 Pathway in the Differentiation of Rat Neuronal Stem Cells' J VET MED SCI. vol. 67, no. 3, 2005, pages 291 - 294, XP055172934

### SEQUENCE LISTING

<110> KEIO UNIVERSITY
   RIKEN
<120> Agent for promoting neurogenesis
<130> 092005
<150> JP2012-103875
   <151> 2012-04-27
<160> 7
<170> PatentIn version 3.4
<210> 1
   <211> 4353
   <212> DNA
   <213> Homo sapiens
<220>
   <221> CDS
   <222> (482)..(1564)
<400> 1
   ttctctcacg aagccccgcc cgcggagagg ttccatattg ggtaaaatct cggctctcgg      60
   agagtcccgg gagctgttct cgcgagagta ctgcgggagg ctcccgtttg ctggctcttg     120
   gaaccgcgac cactggagcc ttagcgggcg cagcagctgg aacgggagta ctgcgacgca     180
   gcccggagtc ggccttgtag gggcgaaggt gcagggagat cgcggcgggc gcagtcttga     240
   gcgccggagc gcgtccctgc ccttagcggg gcttgcccca gtcgcagggg cacatccagc     300
   cgctgcggct gacagcagcc gcgcgcgcgg gagtctgcgg ggtcgcggca gccgcacctg     360
   cgcgggcgac cagcgcaagg tccccgcccg gctgggcggg cagcaagggc cggggagagg     420
   gtgcgggtgc aggcgggggc cccacagggc caccttcttg cccggcggct gccgctggaa     480
   a atg tct cag gag agg ccc acg ttc tac cgg cag gag ctg aac aag aca     529
     Met Ser Gln Glu Arg Pro Thr Phe Tyr Arg Gln Glu Leu Asn Lys Thr
     1               5                   10                  15
   atc tgg gag gtg ccc gag cgt tac cag aac ctg tct cca gtg ggc tct      577
   Ile Trp Glu Val Pro Glu Arg Tyr Gln Asn Leu Ser Pro Val Gly Ser
               20                  25                  30
   ggc gcc tat ggc tct gtg tgt gct gct ttt gac aca aaa acg ggg tta      625
   Gly Ala Tyr Gly Ser Val Cys Ala Ala Phe Asp Thr Lys Thr Gly Leu
           35                  40                  45
   cgt gtg gca gtg aag aag ctc tcc aga cca ttt cag tcc atc att cat      673
   Arg Val Ala Val Lys Lys Leu Ser Arg Pro Phe Gln Ser Ile Ile His
       50                  55                  60
   gcg aaa aga acc tac aga gaa ctg cgg tta ctt aaa cat atg aaa cat      721
   Ala Lys Arg Thr Tyr Arg Glu Leu Arg Leu Leu Lys His Met Lys His
   65                  70                  75                  80
   gaa aat gtg att ggt ctg ttg gac gtt ttt aca cct gca agg tct ctg      769
   Glu Asn Val Ile Gly Leu Leu Asp Val Phe Thr Pro Ala Arg Ser Leu
                   85                  90                  95
   gag gaa ttc aat gat gtg tat ctg gtg acc cat ctc atg ggg gca gat      817
   Glu Glu Phe Asn Asp Val Tyr Leu Val Thr His Leu Met Gly Ala Asp
               100                 105                 110
   ctg aac aac att gtg aaa tgt cag aag ctt aca gat gac cat gtt cag      865
   Leu Asn Asn Ile Val Lys Cys Gln Lys Leu Thr Asp Asp His Val Gln
           115                 120                 125
   ttc ctt atc tac caa att ctc cga ggt cta aag tat ata cat tca gct      913
   Phe Leu Ile Tyr Gln Ile Leu Arg Gly Leu Lys Tyr Ile His Ser Ala
       130                 135                 140
   gac ata att cac agg gac cta aaa cct agt aat cta gct gtg aat gaa      961
   Asp Ile Ile His Arg Asp Leu Lys Pro Ser Asn Leu Ala Val Asn Glu
   145                 150                 155                 160
   gac tgt gag ctg aag att ctg gat ttt gga ctg gct cgg cac aca gat     1009
   Asp Cys Glu Leu Lys Ile Leu Asp Phe Gly Leu Ala Arg His Thr Asp
                   165                 170                 175
   gat gaa atg aca ggc tac gtg gcc act agg tgg tac agg gct cct gag     1057
   Asp Glu Met Thr Gly Tyr Val Ala Thr Arg Trp Tyr Arg Ala Pro Glu
               180                 185                 190
   atc atg ctg aac tgg atg cat tac aac cag aca gtt gat att tgg tca     1105
   Ile Met Leu Asn Trp Met His Tyr Asn Gln Thr Val Asp Ile Trp Ser
           195                 200                 205
   gtg gga tgc ata atg gcc gag ctg ttg act gga aga aca ttg ttt cct     1153
   Val Gly Cys Ile Met Ala Glu Leu Leu Thr Gly Arg Thr Leu Phe Pro
       210                 215                 220
   ggt aca gac cat att aac cag ctt cag cag att atg cgt ctg aca gga     1201
   Gly Thr Asp His Ile Asn Gln Leu Gln Gln Ile Met Arg Leu Thr Gly
   225                 230                 235                 240
   aca ccc ccc gct tat ctc att aac agg atg cca agc cat gag gca aga     1249
   Thr Pro Pro Ala Tyr Leu Ile Asn Arg Met Pro Ser His Glu Ala Arg
                   245                 250                 255
   aac tat att cag tct ttg act cag atg ccg aag atg aac ttt gcg aat     1297
   Asn Tyr Ile Gln Ser Leu Thr Gln Met Pro Lys Met Asn Phe Ala Asn
               260                 265                 270
   gta ttt att ggt gcc aat ccc ctg gct gtc gac ttg ctg gag aag atg     1345
   Val Phe Ile Gly Ala Asn Pro Leu Ala Val Asp Leu Leu Glu Lys Met
           275                 280                 285
   ctt gta ttg gac tca gat aag aga att aca gcg gcc caa gcc ctt gca     1393
   Leu Val Leu Asp Ser Asp Lys Arg Ile Thr Ala Ala Gln Ala Leu Ala
       290                 295                 300
   cat gcc tac ttt gct cag tac cac gat cct gat gat gaa cca gtg gcc     1441
   His Ala Tyr Phe Ala Gln Tyr His Asp Pro Asp Asp Glu Pro Val Ala
   305                 310                 315                 320
   gat cct tat gat cag tcc ttt gaa agc agg gac ctc ctt ata gat gag     1489
   Asp Pro Tyr Asp Gln Ser Phe Glu Ser Arg Asp Leu Leu Ile Asp Glu
                   325                 330                 335
   tgg aaa agc ctg acc tat gat gaa gtc atc agc ttt gtg cca cca ccc     1537
   Trp Lys Ser Leu Thr Tyr Asp Glu Val Ile Ser Phe Val Pro Pro Pro
               340                 345                 350
   ctt gac caa gaa gag atg gag tcc tga gcacctggtt tctgttctgt           1584
   Leu Asp Gln Glu Glu Met Glu Ser
           355                 360
   tgatcccact tcactgtgag gggaaggcct tttcacggga actctccaaa tattattcaa   1644
   gtgcctcttg ttgcagagat ttcctccatg gtggaagggg gtgtgcgtgc gtgtgcgtgc   1704
   gtgttagtgt gtgtgcatgt gtgtgtctgt ctttgtggga gggtaagaca atatgaacaa   1764
   actatgatca cagtgacttt acaggaggtt gtggatgctc cagggcagcc tccaccttgc   1824
   tcttctttct gagagttggc tcaggcagac aagagctgct gtccttttag gaatatgttc   1884
   aatgcaaagt aaaaaaatat gaattgtccc caatcccggt catgcttttg ccactttggc   1944
   ttctcctgtg accccacctt gacggtgggg cgtagacttg acaacatccc acagtggcac   2004
   ggagagaagg cccatacctt ctggttgctt cagacctgac accgtccctc agtgatacgt   2064
   acagccaaaa aggaccaact ggcttctgtg cactagcctg tgattaactt gcttagtatg   2124
   gttctcagat cttgacagta tatttgaaac tgtaaatatg tttgtgcctt aaaaggagag   2184
   aagaaagtgt agatagttaa aagactgcag ctgctgaagt tctgagccgg gcaagtcgag   2244
   agggctgttg gacagctgct tgtgggcccg gagtaatcag gcagccttca taggcggtca   2304
   tgtgtgcatg tgagcacatg cgtatatgtg cgtctctctt tctccctcac ccccaggtgt   2364
   tgccatttct ctgcttaccc ttcacctttg gtgcagaggt ttcttgaata tctgccccag   2424
   tagtcagaag caggttcttg atgtcatgta cttcctgtgt actctttatt tctagcagag   2484
   tgaggatgtg ttttgcacgt cttgctattt gagcatgcac agctgcttgt cctgctctct   2544
   tcaggaggcc ctggtgtcag gcaggtttgc cagtgaagac ttcttgggta gtttagatcc   2604
   catgtcacct cagctgatat tatggcaagt gatatcacct ctcttcagcc cctagtgcta   2664
   ttctgtgttg aacacaattg atacttcagg tgcttttgat gtgaaaatca tgaaaagagg   2724
   aacaggtgga tgtatagcat ttttattcat gccatctgtt ttcaaccaac tatttttgag   2784
   gaattatcat gggaaaagac cagggctttt cccaggaata tcccaaactt cggaaacaag   2844
   ttattctctt cactcccaat aactaatgct aagaaatgct gaaaatcaaa gtaaaaaatt   2904
   aaagcccata aggccagaaa ctccttttgc tgtctttctc taaatatgat tactttaaaa   2964
   taaaaaagta acaaggtgtc ttttccactc ctatggaaaa gggtcttctt ggcagcttaa   3024
   cattgacttc ttggtttggg gagaaataaa ttttgtttca gaattttgta tattgtagga   3084
   atcctttgag aatgtgattc cttttgatgg ggagaaaggg caaattattt taatattttg   3144
   tattttcaac tttataaaga taaaatatcc tcaggggtgg agaagtgtcg ttttcataac   3204
   ttgctgaatt tcaggcattt tgttctacat gaggactcat atatttaagc cttttgtgta   3264
   ataagaaagt ataaagtcac ttccagtgtt ggctgtgtga cagaatcttg tatttgggcc   3324
   aaggtgtttc catttctcaa tcagtgcagt gatacatgta ctccagaggg acagggtgga   3384
   ccccctgagt caactggagc aagaaggaag gaggcagact gatggcgatt ccctctcacc   3444
   cgggactctc cccctttcaa ggaaagtgaa cctttaaagt aaaggcctca tctcctttat   3504
   tgcagttcaa atcctcacca tccacagcaa gatgaatttt atcagccatg tttggttgta   3564
   aatgctcgtg tgatttccta cagaaatact gctctgaata ttttgtaata aaggtctttg   3624
   cacatgtgac cacatacgtg ttaggaggct gcatgctctg gaagcctgga ctctaagctg   3684
   gagctcttgg aagagctctt cggtttctga gcataatgct cccatctcct gatttctctg   3744
   aacagaaaac aaaagagaga atgagggaaa ttgctatttt atttgtattc atgaacttgg   3804
   ctgtaatcag ttatgccgta taggatgtca gacaatacca ctggttaaaa taaagcctat   3864
   ttttcaaatt tagtgagttt ctcaagttta ttatattttt ctcttgtttt tatttaatgc   3924
   acaatatggc attatatcaa tatcctttaa actgtgacct ggcatacttg tctgacagat   3984
   cttaatacta ctcctaacat ttagaaaatg ttgataaagc ttcttagttg tacatttttt   4044
   ggtgaagagt atccaggtct ttgctgtgga tgggtaaagc aaagagcaaa tgaacgaagt   4104
   attaagcatt ggggcctgtc ttatctacac tcgagtgtaa gagtggccga aatgacaggg   4164
   ctcagcagac tgtggcctga gggccaaatc tggcccacca cctgtttggt gtagcctgct   4224
   aagaatggct tttacatttt taaatggttg ggaaagaaaa aaaaagaagt agtagatttt   4284
   gtagcatgtg atgtaagtaa tgtaaaactt aaattccagt atccataaat aaagttttat   4344
   gagaacaga                                                           4353
<210> 2
   <211> 360
   <212> PRT
   <213> Homo sapiens
<400> 2
   Met Ser Gln Glu Arg Pro Thr Phe Tyr Arg Gln Glu Leu Asn Lys Thr
   1               5                   10                  15
   Ile Trp Glu Val Pro Glu Arg Tyr Gln Asn Leu Ser Pro Val Gly Ser
               20                  25                  30
   Gly Ala Tyr Gly Ser Val Cys Ala Ala Phe Asp Thr Lys Thr Gly Leu
           35                  40                  45
   Arg Val Ala Val Lys Lys Leu Ser Arg Pro Phe Gln Ser Ile Ile His
       50                  55                  60
   Ala Lys Arg Thr Tyr Arg Glu Leu Arg Leu Leu Lys His Met Lys His
   65                  70                  75                  80
   Glu Asn Val Ile Gly Leu Leu Asp Val Phe Thr Pro Ala Arg Ser Leu
                   85                  90                  95
   Glu Glu Phe Asn Asp Val Tyr Leu Val Thr His Leu Met Gly Ala Asp
               100                 105                 110
   Leu Asn Asn Ile Val Lys Cys Gln Lys Leu Thr Asp Asp His Val Gln
           115                 120                 125
   Phe Leu Ile Tyr Gln Ile Leu Arg Gly Leu Lys Tyr Ile His Ser Ala
       130                 135                 140
   Asp Ile Ile His Arg Asp Leu Lys Pro Ser Asn Leu Ala Val Asn Glu
   145                 150                 155                 160
   Asp Cys Glu Leu Lys Ile Leu Asp Phe Gly Leu Ala Arg His Thr Asp
                   165                 170                 175
   Asp Glu Met Thr Gly Tyr Val Ala Thr Arg Trp Tyr Arg Ala Pro Glu
               180                 185                 190
   Ile Met Leu Asn Trp Met His Tyr Asn Gln Thr Val Asp Ile Trp Ser
           195                 200                 205
   Val Gly Cys Ile Met Ala Glu Leu Leu Thr Gly Arg Thr Leu Phe Pro
       210                 215                 220
   Gly Thr Asp His Ile Asn Gln Leu Gln Gln Ile Met Arg Leu Thr Gly
   225                 230                 235                 240
   Thr Pro Pro Ala Tyr Leu Ile Asn Arg Met Pro Ser His Glu Ala Arg
                   245                 250                 255
   Asn Tyr Ile Gln Ser Leu Thr Gln Met Pro Lys Met Asn Phe Ala Asn
               260                 265                 270
   Val Phe Ile Gly Ala Asn Pro Leu Ala Val Asp Leu Leu Glu Lys Met
           275                 280                 285
   Leu Val Leu Asp Ser Asp Lys Arg Ile Thr Ala Ala Gln Ala Leu Ala
       290                 295                 300
   His Ala Tyr Phe Ala Gln Tyr His Asp Pro Asp Asp Glu Pro Val Ala
   305                 310                 315                 320
   Asp Pro Tyr Asp Gln Ser Phe Glu Ser Arg Asp Leu Leu Ile Asp Glu
                   325                 330                 335
   Trp Lys Ser Leu Thr Tyr Asp Glu Val Ile Ser Phe Val Pro Pro Pro
               340                 345                 350
   Leu Asp Gln Glu Glu Met Glu Ser
           355                 360
<210> 3
   <211> 3560
   <212> DNA
   <213> Mus musculus
<220>
   <221> CDS
   <222> (399)..(1481)
<400> 3
   gatctggatt cagagcgggg cctccttgga gctgttctcg cgagagttcc gcgagaggct      60
   cccggccgct gcctgtggga tcgccgccac tggagcccaa gcggggcgct gaagcgcgag     120
   cgggtgtctt gcggcgtcgg cgtgcgctcc ctccccgggg agcggctgca ggaggaccgc     180
   ggcgggagca gcctcgagcc gtgcagccgg ctccggcacc ttgccgacgc tcgtaggagc     240
   cgccgcggct gacaggggcg gcgggtcgca gcctccacac ctgcgcgggt ggcgggcgcg     300
   gggtccggtc tgccgcgggc gggcgcagag gagagcgtgc ggctgcaggc aggagccccc     360
   gctcggccac ctcctcgccc cgctgctgcc gctggaag atg tcg cag gag agg ccc     416
                                            Met Ser Gln Glu Arg Pro
                                            1               5
   acg ttc tac cgg cag gag ctg aac aag acc atc tgg gag gtg ccc gaa       464
   Thr Phe Tyr Arg Gln Glu Leu Asn Lys Thr Ile Trp Glu Val Pro Glu
               10                  15                  20
   cga tac cag aac ctg tcc ccg gtg ggc tcg ggc gcc tat ggc tcg gtg       512
   Arg Tyr Gln Asn Leu Ser Pro Val Gly Ser Gly Ala Tyr Gly Ser Val
           25                  30                  35
   tgt gct gct ttt gat aca aag acg ggg cat cgt gtg gca gtt aag aag       560
   Cys Ala Ala Phe Asp Thr Lys Thr Gly His Arg Val Ala Val Lys Lys
       40                  45                  50
   ctg tcg aga ccg ttt cag tcc atc att cac gcc aaa agg acc tac cga       608
   Leu Ser Arg Pro Phe Gln Ser Ile Ile His Ala Lys Arg Thr Tyr Arg
   55                  60                  65                  70
   gag ttg cgt ctg ctg aag cac atg aaa cac gaa aat gtg att ggt ctg       656
   Glu Leu Arg Leu Leu Lys His Met Lys His Glu Asn Val Ile Gly Leu
                   75                  80                  85
   ttg gat gtg ttc aca ccc gca agg tca ctg gag gaa ttc aat gac gtg       704
   Leu Asp Val Phe Thr Pro Ala Arg Ser Leu Glu Glu Phe Asn Asp Val
               90                  95                  100
   tac ctg gtg acc cat ctc atg ggg gcg gac ctg aac aac atc gtg aag      752
   Tyr Leu Val Thr His Leu Met Gly Ala Asp Leu Asn Asn Ile Val Lys
           105                 110                 115
   tgc cag aag ctg acc gac gac cac gtt cag ttt ctc atc tac cag atc      800
   Cys Gln Lys Leu Thr Asp Asp His Val Gln Phe Leu Ile Tyr Gln Ile
       120                 125                 130
   ctc cga ggg ctg aag tat ata cat tcg gct gac ata att cac agg gac      848
   Leu Arg Gly Leu Lys Tyr Ile His Ser Ala Asp Ile Ile His Arg Asp
   135                 140                 145                 150
   cta aag ccc agc aac cta gct gtg aac gaa gac tgt gag ctc aag att      896
   Leu Lys Pro Ser Asn Leu Ala Val Asn Glu Asp Cys Glu Leu Lys Ile
                   155                 160                 165
   ctg gat ttt ggg ctg gct cgg cac act gat gat gag atg aca ggc tac      944
   Leu Asp Phe Gly Leu Ala Arg His Thr Asp Asp Glu Met Thr Gly Tyr
               170                 175                 180
   gtg gct acc agg tgg tac cga gcc cca gag atc atg ctg aat tgg atg      992
   Val Ala Thr Arg Trp Tyr Arg Ala Pro Glu Ile Met Leu Asn Trp Met
           185                 190                 195
   cac tat aac cag aca gtg gat att tgg tcc gtg ggc tgc atc atg gct     1040
   His Tyr Asn Gln Thr Val Asp Ile Trp Ser Val Gly Cys Ile Met Ala
       200                 205                 210
   gag ctg ttg acc gga aga acg ttg ttt cct ggt aca gac cat att aac     1088
   Glu Leu Leu Thr Gly Arg Thr Leu Phe Pro Gly Thr Asp His Ile Asn
   215                 220                 225                 230
   cag ctt cag cag ata atg cgt ctg acg ggg aca ccc cct gct tat ctc     1136
   Gln Leu Gln Gln Ile Met Arg Leu Thr Gly Thr Pro Pro Ala Tyr Leu
                   235                 240                 245
   att aac agg atg cca agc cat gag gca aga aac tac att cag tct ctg     1184
   Ile Asn Arg Met Pro Ser His Glu Ala Arg Asn Tyr Ile Gln Ser Leu
               250                 255                 260
   gcc cag atg ccg aag atg aac ttc gca aat gta ttt att ggt gcc aat     1232
   Ala Gln Met Pro Lys Met Asn Phe Ala Asn Val Phe Ile Gly Ala Asn
           265                 270                 275
   ccc ctg gct gtc gac cta ctg gag aag atg ctc gtt ttg gac tca gat     1280
   Pro Leu Ala Val Asp Leu Leu Glu Lys Met Leu Val Leu Asp Ser Asp
       280                 285                 290
   aag agg atc aca gca gcc caa gct ctt gcg cat gcc tac ttt gct cag     1328
   Lys Arg Ile Thr Ala Ala Gln Ala Leu Ala His Ala Tyr Phe Ala Gln
   295                 300                 305                 310
   tac cac gac cct gat gat gag cct gtt gct gac cct tat gac cag tcc     1376
   Tyr His Asp Pro Asp Asp Glu Pro Val Ala Asp Pro Tyr Asp Gln Ser
                   315                 320                 325
   ttt gaa agc agg gac ctt ctc ata gat gag tgg aag agc ctg acc tat     1424
   Phe Glu Ser Arg Asp Leu Leu Ile Asp Glu Trp Lys Ser Leu Thr Tyr
               330                 335                 340
   gat gaa gtc atc agc ttt gtg cca cca ccc ctt gac caa gaa gaa atg     1472
   Asp Glu Val Ile Ser Phe Val Pro Pro Pro Leu Asp Gln Glu Glu Met
           345                 350                 355
   gag tcc tga gcacctggtt tctgttctgt ctatctcact tcactgtgag          1521
   Glu Ser
       360
   gggaagacct tctcatggga actctccaaa taccattcaa gtgcctcttg ttgaaagatt   1581
   ccttcatggt ggaagggggt gcatgtatgt gttagtgttt gtgtgtgtgt gtgtgtctgt   1641
   ctgttcgtct gtccacctat ctttgtggaa gtcactgtga tggtagtgac tttatgagtt   1701
   gtgaatggtc cttggcagtc tgcctgcttt ctcagagtct gggcaggccg atgggaactg   1761
   tcatctcctt agggatgtgt gtgttcagtg caaagtaaga aatatgaaaa tatccctgtt   1821
   cttagttacc ttgccacttt ggcttctcct gtggccctgc ctttaccata tcagtgacag   1881
   agagaggctg cttcaggtct gaggctatcc ctcagccatg cataaagtcc aagagaacca   1941
   actggctcct ggtctctagc ctgtgaccgg cttgcttaat gtcctcagaa cctgacaggt   2001
   atgttcaaaa ctgtcagtct gtttgtgcct taaaagggtg agaagggcgc gtagatagtt   2061
   acagagtctc agctgctgac gttctgagcc aggcaagtgc acggggctgt tggatggcca   2121
   gtggggagct ggaaaaaaca aggcagcctt taggaaggcc atggtgcatg tgtgtgcatg   2181
   cgtgtatgtg cagccgccct ccctcacttc aggagcaagc tgtttgctgt gcttaccctt   2241
   cacctcagtg cagaggtctc cagtgccgag cacaggcacc tgccatcagt agttcctgtg   2301
   tcatcttcac atctagcaga gcacggatgt gtttgcatgc tgtgctcttg gagcttgtcc   2361
   tgtcttctgg aagccctgga caaggcgtgt gaaggcttcc cagaagttcc tgtccacatt   2421
   gcctccgccc accgacgcca tgggcacact gctccctcct cctcctccag ctactttgtg   2481
   ttgaacacaa ttgattctcc aggtgctcat ggtgcaggaa aacaggacag acagagagca   2541
   cctgaaccct tgccatctga tgtcaccaat tcaggaaaac gagtcctctc ctaggactat   2601
   ccccggttct ggaaatcatg ttctcctcac tcatggtgac aagctaagaa agctgaacaa   2661
   agggagagac gagagcgcct gaagccagga gctcctttac tatctttctc aaaagggttg   2721
   ttagacacaa accaagtcat caaggccccg ctcctctcct cggaagggtc ccccaccccc   2781
   cggcagcttg acactgaatc cagtgtcaat ttggggagaa agcagttttg tcttggaatt   2841
   ttgtatgttg taggaatcct tagagagtgt ggttccttct gatggggaga aagggcaaat   2901
   tattttaata ttttgtattt tcacctttat aaacatgaat cctcaggggt gaagaactgt   2961
   ttgcataatt ttctgaattt tgagcacttt gtgctatata aggacccata tttaagcttt   3021
   gtgtgcagta agaaagtgta aagccaattc cagtgttgga cgtgacaggt cttgtgttta   3081
   ggtcaaggtg tctcctctca gtgcagggac atgcctgctc tgtggggcag gcgaggaccc   3141
   tgaatcattt ggagcccaga aggaggcaga ctggccaggt ctcaccacct cagtgtgcag   3201
   ttcaactcca tgccatccca tcaagatggg ttagtagcag tgtctgtttt tgaatgccaa   3261
   gtgtgatttc caacaattct gctctggtta tttcattgaa gacatctttg cacatgtgac   3321
   catgctgtgt taggggctgt gttccaggga ctggactcga agctagaact ggcagaagag   3381
   ttctggcatc cacagcgcaa tgctgccacc acccagtttc ttcatcagaa gacaagggaa   3441
   cgagaaaact gctgttcgtt tgtatttgtg aacttggctg taatctggta tgccatagga   3501
   tgtcagataa taccactggt taaaataaag cctagttttc aaattcaaaa aaaaaaaaa    3560
<210> 4
   <211> 360
   <212> PRT
   <213> Mus musculus
<400> 4
   Met Ser Gln Glu Arg Pro Thr Phe Tyr Arg Gln Glu Leu Asn Lys Thr
   1               5                   10                  15
   Ile Trp Glu Val Pro Glu Arg Tyr Gln Asn Leu Ser Pro Val Gly Ser
               20                  25                  30
   Gly Ala Tyr Gly Ser Val Cys Ala Ala Phe Asp Thr Lys Thr Gly His
           35                  40                  45
   Arg Val Ala Val Lys Lys Leu Ser Arg Pro Phe Gln Ser Ile Ile His
       50                  55                  60
   Ala Lys Arg Thr Tyr Arg Glu Leu Arg Leu Leu Lys His Met Lys His
   65                  70                  75                  80
   Glu Asn Val Ile Gly Leu Leu Asp Val Phe Thr Pro Ala Arg Ser Leu
                   85                  90                  95
   Glu Glu Phe Asn Asp Val Tyr Leu Val Thr His Leu Met Gly Ala Asp
               100                 105                 110
   Leu Asn Asn Ile Val Lys Cys Gln Lys Leu Thr Asp Asp His Val Gln
           115                 120                 125
   Phe Leu Ile Tyr Gln Ile Leu Arg Gly Leu Lys Tyr Ile His Ser Ala
       130                 135                 140
   Asp Ile Ile His Arg Asp Leu Lys Pro Ser Asn Leu Ala Val Asn Glu
   145                 150                 155                 160
   Asp Cys Glu Leu Lys Ile Leu Asp Phe Gly Leu Ala Arg His Thr Asp
                   165                 170                 175
   Asp Glu Met Thr Gly Tyr Val Ala Thr Arg Trp Tyr Arg Ala Pro Glu
               180                 185                 190
   Ile Met Leu Asn Trp Met His Tyr Asn Gln Thr Val Asp Ile Trp Ser
           195                 200                 205
   Val Gly Cys Ile Met Ala Glu Leu Leu Thr Gly Arg Thr Leu Phe Pro
       210                 215                 220
   Gly Thr Asp His Ile Asn Gln Leu Gln Gln Ile Met Arg Leu Thr Gly
   225                 230                 235                 240
   Thr Pro Pro Ala Tyr Leu Ile Asn Arg Met Pro Ser His Glu Ala Arg
                   245                 250                 255
   Asn Tyr Ile Gln Ser Leu Ala Gln Met Pro Lys Met Asn Phe Ala Asn
               260                 265                 270
   Val Phe Ile Gly Ala Asn Pro Leu Ala Val Asp Leu Leu Glu Lys Met
           275                 280                 285
   Leu Val Leu Asp Ser Asp Lys Arg Ile Thr Ala Ala Gln Ala Leu Ala
       290                 295                 300
   His Ala Tyr Phe Ala Gln Tyr His Asp Pro Asp Asp Glu Pro Val Ala
   305                 310                 315                 320
   Asp Pro Tyr Asp Gln Ser Phe Glu Ser Arg Asp Leu Leu Ile Asp Glu
                   325                 330                 335
   Trp Lys Ser Leu Thr Tyr Asp Glu Val Ile Ser Phe Val Pro Pro Pro
               340                 345                 350
   Leu Asp Gln Glu Glu Met Glu Ser
           355                 360
<210> 5
   <211> 23
   <212> RNA
   <213> Mus musculus
<400> 5
   caaagugcuu acagugcagg uag 23
<210> 6
   <211> 23
   <212> RNA
   <213> Mus musculus
<400> 6
   caaagugcua acagugcagg uag 23
<210> 7
   <211> 21
   <212> RNA
   <213> Mus musculus
<400> 7
   uaaagugcug acagugcaga u 21

## Description

The present invention relates to an agent for use in a method of promoting neuronal differentiation of neural stem cells.

### Background Art

Central nervous system (CNS) injury and disease have gained new prominence in modern medicine. Recent progress in stem cell biology, typified by induced pluripotent stem cell (iPSC) technology, has drawn attention to stem cells as innovative resources for transplantation therapy and individualized drug screening. Multipotent neural stem cells (NSCs), which give rise to all types of neural cells within the CNS, are now readily obtained from iPSCs. However, the specific and efficient induction of homogeneous target cell populations from NSCs remains difficult due to the complex mechanisms regulating NSC development and differentiation. Therefore, further identification of the means by which specific cell types are generated from NSCs is urgently required to facilitate their therapeutic application.

The present inventors have been using a recently-developed embryonic stem cell (ESC)-derived neurosphere culture system to investigate the molecular mechanisms governing NSC differentiation (non-patent document 1, patent document 1). Although NSCs possess the multipotency to differentiate into neurons and glial cells, neurogenesis largely precedes gliogenesis during CNS development in vertebrates. This neurogenesis-to-gliogenesis switch requires the temporal identity transition of NSCs (non-patent document 2). Importantly, the neurosphere culture system developed by the present inventors can recapitulate neural development in vivo, including this temporal regulation. Using this system, the present inventors previously discovered that Coup-tfI and II (Coup-tfs; also known as Nr2f1 and Nr2f2) act as critical molecular switches for the differentiation potential transition of NSCs (non-patent document 1, patent document 1).

Patent Document 2 relates to the treatment of traumatic brain injury and Alzheimer's disease using p38 inhibitors.

Patent Document 3 relates to the treatment of traumatic brain injury, cerebral ischemia, Huntingdon's disease and Alzheimer's disease using p38 inhibitors.

Patent Document 4 relates to the treatment of nerve system diseases such as cerebral infarction, traumatic brain injury, cerebral ischemia, Huntingdon's disease and Alzheimer's disease using p38 siRNA.

Patent Document 5 relates to the treatment of nerve system diseases such as, traumatic brain injury, brain ischemia, Huntingdon's disease and Alzheimer's disease using p38 inhibitors.

Patent Document 6 relates to the treatment of CNS injury such as cerebral infarction, traumatic brain injury, cerebral ischemia, Huntingdon's disease and Alzheimer's disease using p38 inhibitors.

Patent Document 7 relates to a method of reducing amyloid plaque burden using p38 inhibitors.

Patent Document 8 relates to the treatment of working memory impairment due to aging, Alzheimer's disease, amyotrophic sclerosis (ALS), Parkinson's disease, alcoholism, alcohol withdrawal, or Huntingdon's disease using p38 inhibitors.

Non-patent documents 3-5 relate to the use of p38 inhibitors in *in vitro* methods for promoting neuronal cell differentiation in neuronal stem cells and/or converting gliogenic neuronal stem cells to neurogenic stem cells.

### [Document List]

### [patent document]

patent document 1: WO2010/018652
patent document 2: WO05/039393
patent document 3: WO01/01986
patent document 4: WO2010/018652
patent document 5: WO00/17175
patent document 6: WO00/12497
patent document 7: WO2012/154814
patent document 8: US2012/245188

### [non-patent documents]

non-patent document 1: Nat. Neurosci. 11, 1014-1023 (2008)
non-patent document 2: Nat. Neurosci. 9, 743-751 (2006)
non-patent document 3: J. Cell. Biochem. 2001, 83(4), 585
non-patent document 4: J. Clin. Rehab Tissue Eng. Res. 2007, 11(37), 7329
non-patent document 5: Neurosci. Res. 2003, 47(2), 209

### SUMMARY OF THE INVENTION

### Problems to be Solved by the Invention

An object of the present study is to elucidate the molecular mechanism involved in the competence change of NSCs and provide a technique for improving the differentiation efficiency of neural stem cells into neurons.

### Means of Solving the Problems

The present inventors have conducted intensive studies in an attempt to solve the aforementioned problems and identified that micro RNA-17 (miR-17)/106-p38 pathway is an effector of transition of neural stem cells from a neurogenic type to a gliogenic type. Overexpression of miR-17 inhibited acquisition of gliogenic competence, and restored the neuropotency of neural stem cells without accompanying changes in the epigenetic status of glial marker gene promoter, in NSCs in which developmental stage has progressed up to the period when NSCs are generally differentiated into gliogenic NSCs. They have further identified that the direct target of miR-17/106 is p38. Restoration of neuropotency by p38 inhibition was achieved in mouse and human NSCs. They have conducted further studies based on these findings and completed the present invention.

Accordingly, the present invention relates to the following.
[1] A p38 inhibitor selected from miR-17, miR-106a and miR-106b for use in a method of treating or preventing a disorder selected from cerebral infarction, traumatic brain injury, cerebral ischemia, Huntington's disease and Alzheimer's disease.
[2] An agent comprising the p38 inhibitor according to 1 and a pharmaceutically acceptable carrier for use according to 1.
[3] An *in vitro* method of promoting neuronal differentiation of neural stem cells, comprising cultivating neural stem cells in the presence of the p38 inhibitor as defined in 1.
[4] The method according to 3, wherein the neural stem cells are gliogenic.
[5] The method according to 3, wherein the neural stem cells are neurogenic.
[6] An *in vitro* method of converting a gliogenic neural stem cell to a neurogenic neural stem cell, comprising cultivating gliogenic neural stem cells in the presence of the p38 inhibitor as defined in 1.

### Effect of the Invention

According to the present invention, differentiation of a neural stem cell into a neuron can be promoted by a new mechanism of p38 suppression. This results from the restoration of neurogenic competence in gliogenic neural stem cells, and neuronal differentiation can be induced exclusively.

### Brief Description of the Drawings

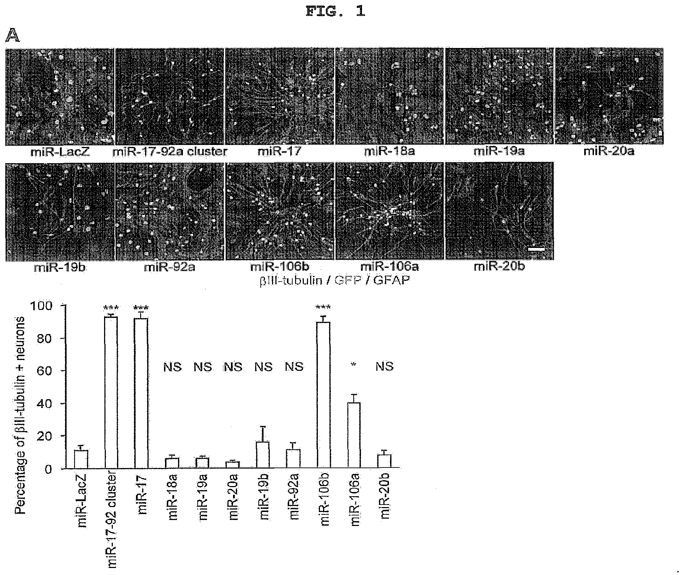

Fig. 1 shows representative immunocytochemical images of differentiated p2 neurospheres derived from NSCs infected with lentiviruses expressing microRNAs and GFP at the primary (p0) neurosphere stage. MiR-LacZ microRNA was used as a control. Neurospheres were immunostained with antibodies against βIII-tubulin, GFAP and GFP. Scale bar, 50 µm. The graph shows the percentage of βIII-tubulin-positive (βIII-tubulin +) neurons among virus-infected cells differentiated from p2 neurospheres (t test, n (≥ 100 cells) = 3, *P < 0.05 and ***P < 0.001). Bars and error bars represent the mean+s.e.m for each condition.

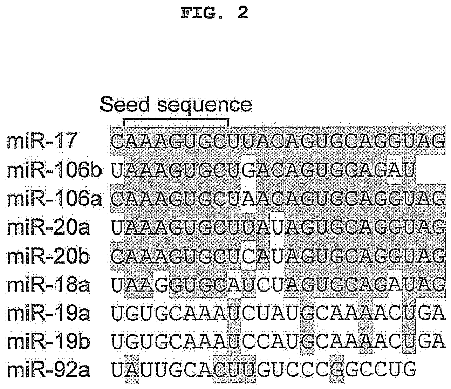

Fig. 2 shows the sequence comparison of microRNAs encoded by the miR-17-92, miR-106b-25 and miR-106a-363 clusters. The nucleotides identical to the corresponding nucleotides in miR-17 are shaded.

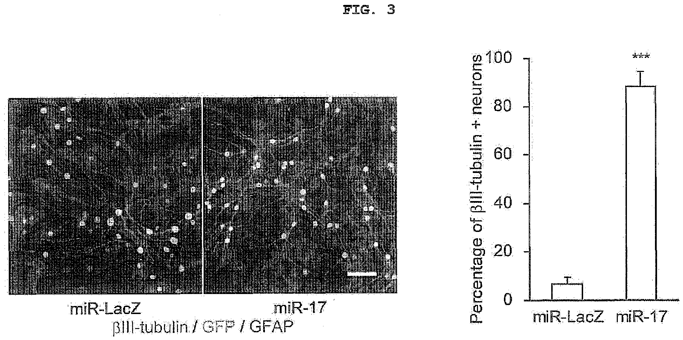

Fig. 3 shows immunocytochemical images of miR-17 overexpressing p2 neurospheres. MiR-17-overexpressing p2 neurospheres did not undergo gliogenesis in response to LIF (10 ng/ml) and BMP2 (100 ng/ml). The graph shows the percentage of βIII-tubulin-positive (βIII-tubulin +) neurons among virus-infected cells differentiated from p2 neurospheres (t test, n (≥ 100 cells) = 3, ***P < 0.001). Scale bar, 50 µm. Bars and error bars represent the mean+s.e.m for each condition.

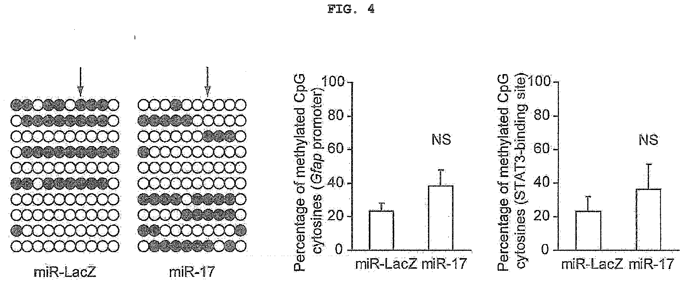

Fig. 4 shows the analysis results of the CpG methylation status of the Gfap promoter by bisulfite sequencing. The methylation frequency of 10 CpG sequences on the Gfap promoter, including the CpG site in the STAT3-binding sequence as indicated by the arrows, was examined. The graphs show the percentages of methylated CpG cytosines in the whole Gfap promoter (left graph) and the STAT3-binding region (right graph) (t test, n (≥ 10 clones) = 3, NS (not significant), P> 0.05). Bars and error bars represent the mean+s.e.m for each condition.

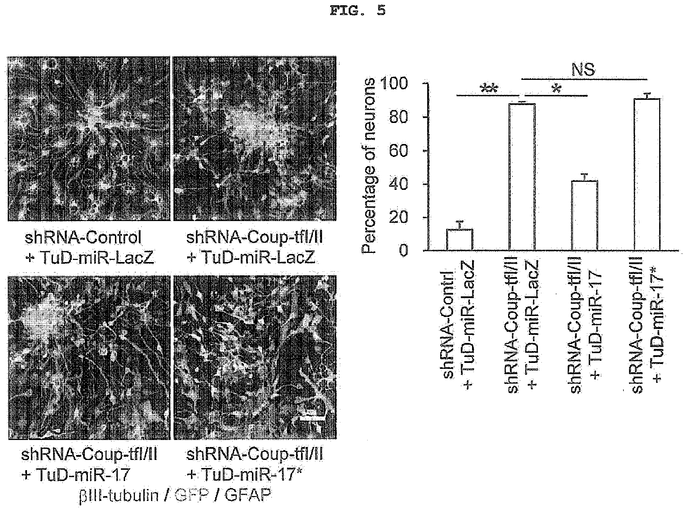

Fig. 5 shows immunocytochemical images of the cells differentiated from Coup-tfs-knockdown, miR-17-suppressed p0 neurospheres. Overexpression of TuD-miR-17 (GFP-positive cells) cancelled the neuronal differentiation-promoting action in Coup-tfs knockdown p2 neurospheres and restored differentiation into astrocytes as assessed by reduction in βIIItubulin-positive nerve cells and increased GFP immunostaining. The graph shows the percentage of neurons (t test, n (≥ 100 cells) = 3, **, *P < 0.05). Scale bar, 50 µm. Bars and error bars represent the mean+s.e.m for each condition.

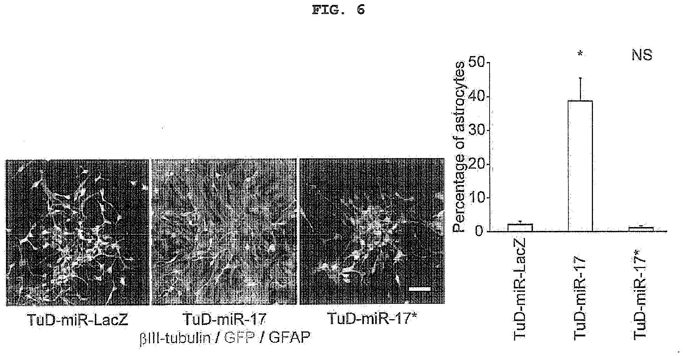

Fig. 6 shows immunocytochemical images of the cells differentiated from TuD-miR-17 overexpressing p0 neurospheres in the presence of glial differentiation inducing factors. Overexpression of TuD-miR-17 caused precocious gliogenesis from p0 neurospheres in the presence of LIF and BMP2. The graph shows the percentage of astrocytes (t test, n (≥ 100 cells) = 3, *P < 0.05). Scale bar, 50 µm. Bars and error bars represent the mean+s.e.m for each condition.

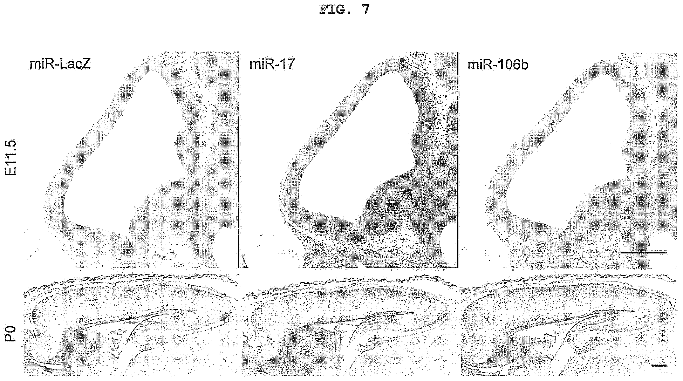

Fig. 7 shows the expression of miR-17 and miR-106b at E11.5 and P0 which was determined by in situ hybridization with specific locked nucleic acid (LNA) probes. Scale bar, 300 µm.

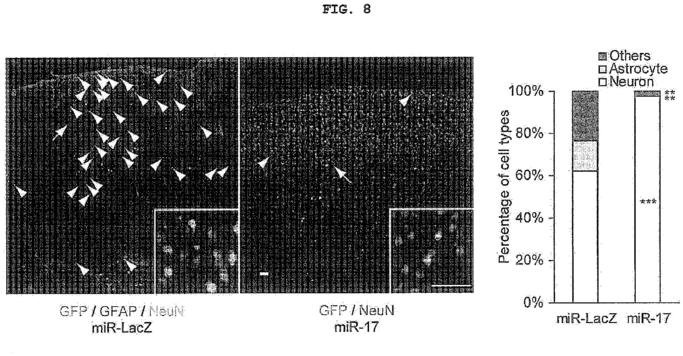

Fig. 8 shows immunocytochemical images of cerebral cortex sections of a mouse injected into the cerebral ventricle with lentiviruses for the overexpression of miR-LacZ or miR-17. The lentiviruses for the overexpression of miR-LacZ or miR-17 were injected in utero into the cerebral ventricle of mouse embryos at E10.5, and the fates of the infected cells were examined in the cerebral cortex at P30 (t test, n (≥ 250 cells, ≥ three independent sections) = three independent embryos, **P < 0.01 and ***P < 0.001). Neuronal nuclei were stained with an antibody against NeuN. Arrowheads indicate non-neuronal cells, which include GFAP-positive astrocytes. Most GFAP-negative non-neuronal cells ('Others') appeared to be immature astrocytes. Higher magnification images of the cells indicated with arrows are shown as inserts. Scale bar, 50 µm.

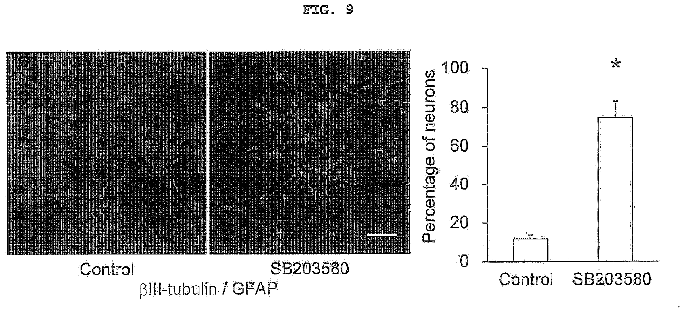

Fig. 9 shows immunocytochemical images of the cells differentiated from SB203580-treated p2 neurospheres. Treatment with the p38 inhibitor SB203580 during their growth increased the percentage of neurons differentiated from p2 neurospheres (t test, n (≥ 150 cells) = 3, *P < 0.05). Scale bar, 50 µm. Bars and error bars represent the mean+s.e.m for each condition.

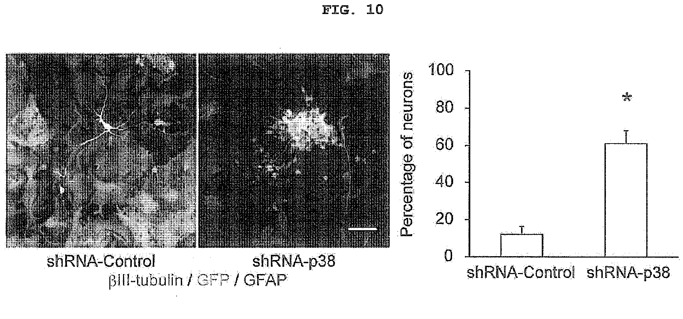

Fig. 10 shows immunocytochemical images of the cells differentiated from p2 neurospheres introduced with p38 specific shRNA. The p38-specific shRNA introduced by lentiviral infection increased the neuropotency of p2 neurospheres (t test, n (≥ 100 cells) = 3, *P < 0.05). Scale bar, 50 µm. Bars and error bars represent the mean+s.e.m for each condition.

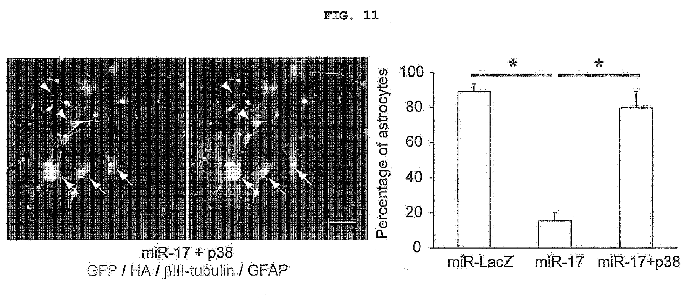

Fig. 11 shows immunocytochemical images of the cells differentiated from p2 neurospheres overexpressing miR-17 and miR-17s-resistant p38 mRNA. Simultaneous overexpression of miR-17 and miR-17s-resistant p38 mRNA (yielding 3x HA-tagged N terminal p38 as the protein product) eliminated the effect of overexpressed miR-17 to promote neuronal differentiation in p2 neurospheres. Hence, most of the progenitor cells that exogenously express p38 were fated to become astrocytes once again (t test, n (≥ 150 cells) = 3, *P < 0.05). Arrows indicate GFP, HA-tagged p38 and GFAP triple-positive astrocytes. Arrowheads indicate GFP and βIII-tubulin double-positive neurons. Scale bar, 50 µm. Bars and error bars represent the mean+s.e.m for each condition.

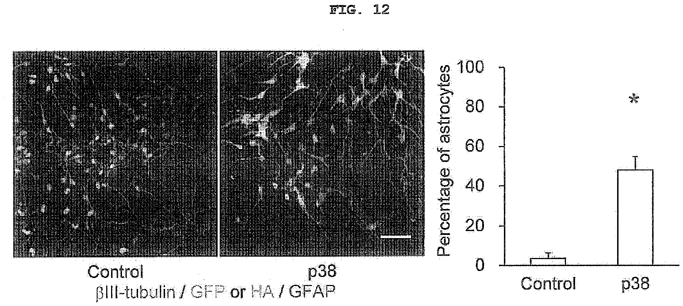

Fig. 12 shows immunocytochemical images of the cells differentiated from p38 overexpressing p0 neurospheres in the presence of a glial differentiation inducing factors. Overexpression of HA-tagged p38 caused precocious gliogenesis from p0 neurospheres in the presence of LIF and BMP2 (t test, n (≥ 100 cells) = 3, *P < 0.05). Scale bar, 50 µm. Bars and error bars represent the mean+s.e.m for each condition. Bars and error bars represent the mean+s.e.m for each condition.

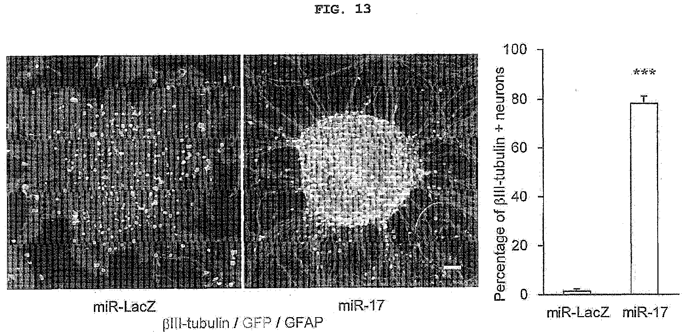

Fig. 13 shows immunocytochemical images of the cells differentiated from miR-17-overexpressing p3 neurospheres. Overexpression of miR-17 restored neuropotency of mouse p3 neurospheres (t test, n (≥ 100 cells) = 3, ***P < 0.001). Scale bar, 50 µm.

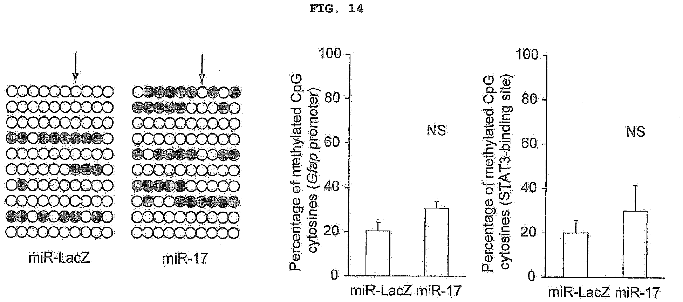

Fig. 14 shows the analysis results of the CpG methylation status of the Gfap promoter by bisulfite sequencing. The graphs show the percentages of methylated CpG cytosines in the whole Gfap promoter (left graph) and the STAT3-binding region (right graph) (t-test, n (≥ 10 clones) = 3, NS, P > 0.05).

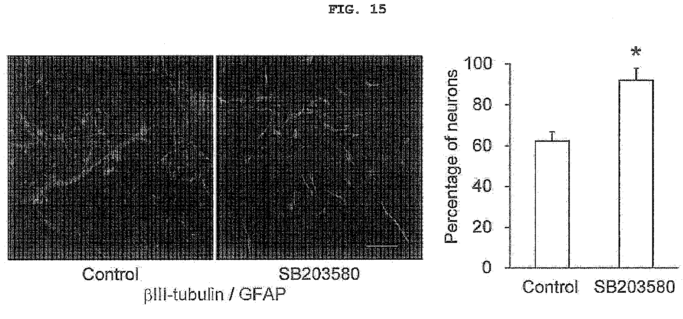

Fig. 15 shows immunocytochemical images of the cells differentiated from human neurospheres treated with SB203580. Treatment of human neurospheres with the p38 inhibitor, SB203580, increased the percentage of neurons differentiated from the neurospheres (t-test, n (≥ 150 cells) = 3, *P < 0.05). Scale bar, 50 µm.

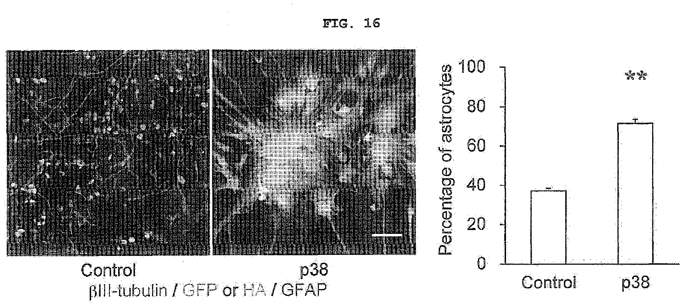

Fig. 16 shows immunocytochemical images of the cells differentiated from human neurosphere overexpressing p38, in the presence of a glial differentiation inducing factors. Overexpression of HA-tagged p38 in human neurospheres increased the percentage of astrocytes differentiated from the neurospheres in the presence of LIF and BMP2 (t test, n (≥ 100 cells) = 3, *P < 0.05). Scale bar, 50 µm. Bars and error bars represent the mean+s.e.m for each condition.

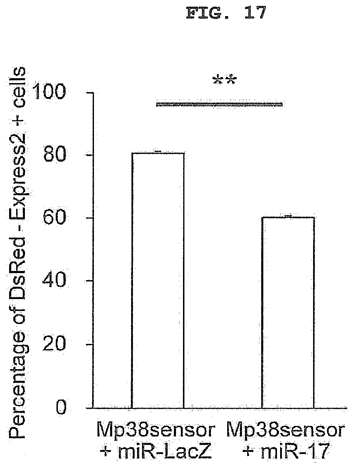

Fig. 17 is a graph showing that miR-17 binds to a binding site in the 3' untranslated region (UTR) of mouse p38 mRNA. To confirm direct interaction between miR-17 and its binding site in 3'UTR of p38 mRNA, a reporter assay was performed. The miR-17 binding site in 3'UTR of p38 mRNA (to be referred to as "Mp38 sensor"; 2970-2992 of NM_011951) was cloned into a mammal expression vector of the DsRed-Express2 reporter system. After transformation of HEK293 cells, lentiviruses expressing miR-LacZ or miR-17 were introduced into a stable transformant. One week later, the intracellular expression level of DsRed-Express2 was analyzed by FACS. Forced expression of miR-17 caused a decrease in DsRed-Express2 linked to the Mp38 sensor, as compared to miR-LacZ. (t-test, n (≥ 3000 cells) = 3, **P < 0.01). Bars and error bars represent the mean+s.e.m for each condition.

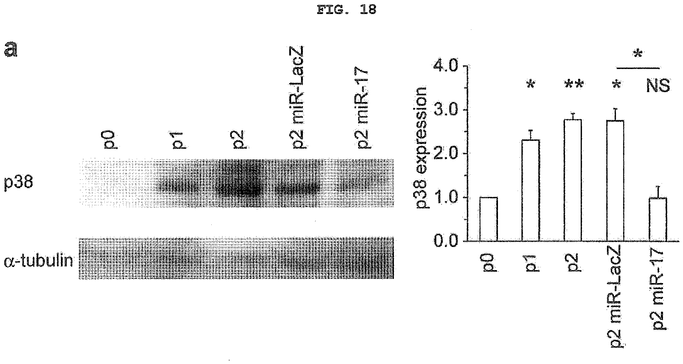

Fig. 18 shows the comparison results of p38 expression by Western blotting. The expression levels of p38 in p0, p1 and p2 neurospheres were examined by an anti-p38 antibody (Cell Signaling Technology 9212). miR-17 suppressed p38 expression at p2 stage (t-test, n = 3, NS (not significant) p > 0.05, *p < 0.05 and ** p < 0.01; p1 *p = 0.0281, p2 **p = 0.0063, p2 miR-LacZ *p = 0.0243, p2 miR-17 p = 0.9704, *p =0.0393).

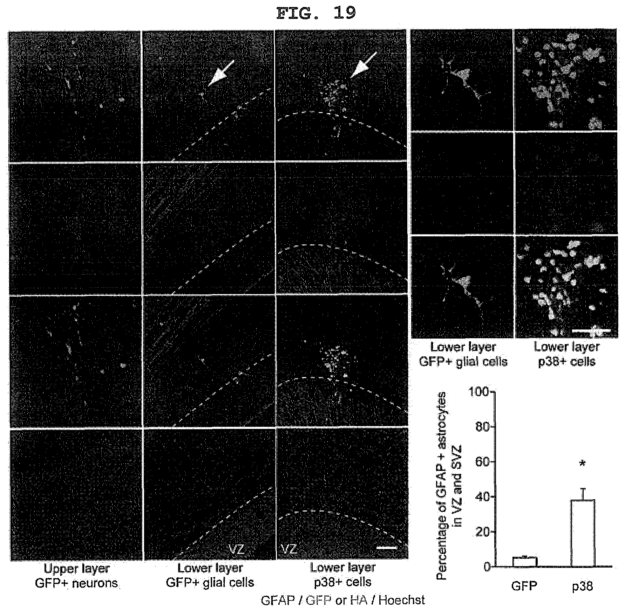

Fig. 19 shows the results indicating that p38 controls acquisition of gliogenic competence in the developing mouse forebrain. Overexpression of p38 resulted in the production of cell clusters having abnormal early expression of GFAP protein in VZ and SVZ (t-test, n (≥ 100 cells) = 5 independent embryos, *p<0.05; *p=0.0284) (left Figure). Higher magnification images of the cells indicated with arrows are shown in the right Figure. In the left and right Figures, the photographs of the uppermost panel indicate GFP or HA, the photographs in the second panel from the top indicate GFAP, and the photographs in the third panel from the top show merged image of GFAP, and GFP or HA. The lowermost panel of the left Figure shows Hoechst staining. Scale bar: 50 µm. In the lower right graph, bars and error bars represent the mean+s.e.m.

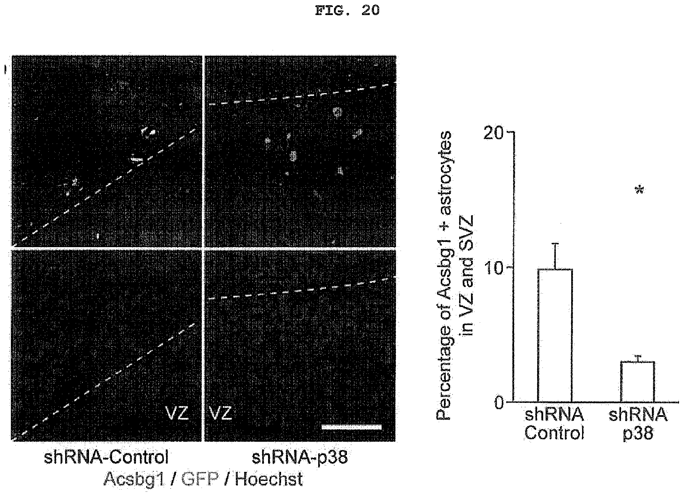

Fig. 20 shows the results indicating that p38 controls acquisition of gliogenic competence in the developing mouse forebrain. The photograph of the upper panel of the Figure shows merged image of Acsbg1 and GFP, and the photograph of the lower panel shows Hoechst staining. The percentage of Acsbg1-expressing astrocytes decreased in VZ and SVZ due to shRNA-mediated knockdown of p38 (t-test, n (≥ 100 cells) = 5 independent embryos, *p<0.05;*p=0.0158). Scale bar: 50 µm. Bars and error bars represent the mean+s.e.m in the graph.

### Description of Embodiments

The present invention provides a p38 inhibitor selected from miR-17, miR-106a and miR-106b for use in a method of treating or preventing a disorder selected from cerebral infarction, traumatic brain injury, cerebral ischemia, Huntington's disease and Alzheimer's disease, as well as an agent comprising this p38 inhibitor and a pharmaceutically acceptable carrier for the same use.

A p38 inhibitor means a substance that suppresses expression or function of p38.

p38, also called Mapk14 (mitogen-activated protein kinase 14), is a member of the MAP kinase family, and is a known protein kinase activated by stimulation by cytokine, UV irradiation, heat or osmotic pressure stress

In the present specification, p38 generally means one derived from a mammal. Examples of the mammal include rodents such as mouse, rat, and guinea pigs, experiment animals such as rabbits, domestic animals such as swine, bovine, goat, horse, and mink, pets such as dogs and cats, primates such as human, monkey, Macaca fascicularis, Macaca mulatta, marmoset, orangutans and chimpanzees. p38 is preferably derived from primates (e.g. humans) or rodents (e.g. mice).

In the present specification, factors such as polypeptide, polynucleotide "derived from organism X" means that the polypeptide or polynucleotide has an amino acid sequence or polynucleotide sequence the same as or substantially the same as that of the polypeptide or polynucleotide naturally expressed in the organism X. Being "substantially the same" means that the interested amino acid sequence or nucleic acid sequence has 70% or more (preferably 80% or more, more preferably 90% or more, further preferably 95% or more, most preferably 99% or more) identity with the amino acid sequence or nucleic acid sequence of a factor naturally expressed in organism X, and maintains the activity of the protein.
The amino acid sequence and nucleotide sequence of p38

The amino acid sequence and nucleotide sequence of p38 are known. The representative amino acid sequences of human p38 and mousep 38 and the polynucleotide sequences (cDNA or mRNA sequence) encoding same are as described below. In the parentheses, the accession numbers of GENBANK provided by NCBI are shown.
[human p38]
   amino acid sequence: accession number NP_001306 (version NP_001306.1) (SEQ ID NO: 2)
   nucleotide sequence (cDNA sequence): accession number NM_001315 (version NM_001315.2) (SEQ ID NO: 1)
[mouse p38]
   amino acid sequence: accession number NP_001161980 (version NP_001161980.1) (SEQ ID NO: 4)
   nucleotide sequence (cDNA sequence): accession number NM_001168508 (version NM_001168508.1) (SEQ ID NO: 3)

In the present specification, nucleotide sequences are described as DNA sequences unless otherwise specified; however, when the polynucleotide is an RNA, thymine (T) should read as uracil (U) as appropriate.

Examples of the function of p38 include phosphorylation of serine or treonine in a substrate protein such as ATF2, MEF2C, MAX, CDC25B, and p53.

Examples of the p38 inhibitor include a nucleic acid which hybridizes, under physiological conditions, to a DNA or mRNA encoding p38, thereby inhibiting the transcription and/or translation thereof, an expression vector of the nucleic acid, or a low-molecular-weight compound.

Examples of the nucleic acid which hybridizes, under physiological conditions, to a DNA or mRNA encoding p38, thereby inhibiting the transcription and/or translation thereof is micro RNA(miRNA) targeting p38, with siRNA a precursor to micro RNA, an antisense nucleic acid also being disclosed herein.

Examples of the siRNA targeting p38 also disclosed herein include
(A) an RNA comprising a nucleotide sequence complementary to the nucleotide sequence of an mRNA (mature mRNA or primary transcript) that encodes p38 or a partial sequence thereof having a length of 18 bases or more; and
(B) an RNA which comprises a nucleotide sequence having a length of 18 bases or more and capable of specifically hybridizing to an mRNA (mature mRNA or primary transcript) that encodes p38 in a cell of the target animal (preferably human or mouse), and suppresses transcription and/or translation of p38 by the hybridization.

In the present specification, "specific hybridization" means that a nucleic acid more strongly hybridizes to the target nucleotide than other nucleotides.

Examples of the nucleotide sequence of mRNA encoding p38 include the nucleotide sequence shown by SEQ ID NO: 1 (human p38), and the nucleotide sequence shown by SEQ ID NO: 3 (mouse p38) .

What is called RNA interference (RNAi), a phenomenon in which transferring siRNA of a short double-stranded RNA and the like into a cell results in the degradation of mRNAs that are complementary to the RNA, has long been known to occur in nematodes, insects, plants and the like; recently, this phenomenon was confirmed as occurring also in animal cells [Nature, 411(6836): 494-498 (2001)], and this is attracting attention as a ribozyme-substitute technique.

A representative siRNA is a double-stranded oligo-RNA consisting of an RNA having a sequence complementary to the nucleotide sequence of the mRNA of the target gene or a partial sequence thereof (hereinafter, target nucleotide sequence) and a complementary strand for the RNA. A single-stranded RNA wherein a sequence complementary to the target nucleotide sequence (first sequence) and a complementary sequence for the same (second sequence) are linked via a hairpin loop portion, and wherein the first sequence forms a double-stranded structure with the second sequence by assuming a hairpin loop structure (small hairpin RNA: shRNA), also represents a preferred embodiment of siRNA.

The length of the portion complementary to the target nucleotide sequence, contained in the siRNA, is normally about 18 bases or more, preferably 19 bases or more, more preferably about 21 bases or more, and is not particularly limited as long as the expression of the target gene can be specifically suppressed. When the siRNA is longer than 23 bases, the siRNA undergoes degradation in cells to produce an siRNA of about 20 bases long; therefore, theoretically, the upper limit of the portion complementary to the target nucleotide sequence is the full length of the nucleotide sequence of an mRNA (mature mRNA or primary transcript) of the target gene. However, in view of the avoidance of interferon induction, the ease of synthesis, antigenicity problems and the like, the length of the complementary portion is, for example, about 50 bases or less, preferably about 25 bases or less, most preferably about 23 bases or less. Hence, the length of the complementary portion is, for example, normally, about 18-50 bases, preferably about 19 to about 25 bases, more preferably about 21 to about 23 bases.

In addition, the length of each RNA chain constituting siRNA is also disclosed herein generally about 18 bases or more, preferably 19 bases or more, more preferably about 21 bases or more, and is not particularly limited as long as the expression of the target gene can be specifically suppressed. Theoretically, the length of each RNA chain does not have an upper limit. However, in view of the avoidance of interferon induction, the ease of synthesis, antigenicity problems and the like, the length of the siRNA is, for example, about 50 bases or less, preferably about 25 bases or less, most preferably about 23 bases or less. Hence, the length of each siRNA is, for example, about 18 to 50 bases, preferably about 19 to about 25 bases, more preferably about 21 to about 23 bases. The length of the shRNA is expressed as the length of the double-stranded moiety when the shRNA provides a double-stranded structure.

It is preferable that the target nucleotide sequence and the sequence complementary thereto contained in the siRNA also disclosed herein be completely complementary to each other. However, in the presence of a base mutation at a position apart from the center of the siRNA (the mutation can be fall in the range of identity of at least 90% or more, preferably 95% or more), the cleavage activity by RNA interference is not completely lost, but a partial activity can remain. On the other hand, a base mutation in the center of the siRNA has a major influence to the extent that can extremely reduce the mRNA cleavage activity by RNA interference.

The siRNA also disclosed herein may have an additional base that does not form a base pair at the 5'- and/or 3'-end. The length of the additional base is not particularly limited as long as siRNA can specifically suppress the expression of the target gene, and is normally 5 bases or less, for example, 2 to 4 bases. Although the additional base may be a DNA or an RNA, use of a DNA makes it possible to improve the stability of the siRNA. Examples of the sequences of such additional bases include, but are not limited to, the sequences ug-3', uu-3', tg-3', tt-3', ggg-3', guuu-3', gttt-3', ttttt-3', and uuuuu-3'.

The length of the loop portion of the hairpin loop of the shRNA also disclosed herein is not particularly limited, as long as the expression of the target gene can be specifically suppressed, and the length is normally about 5 to 25 bases. The nucleotide sequence of the loop portion is not particularly limited as long as a loop can be formed, and shRNA can specifically suppress the expression of the target gene.

Examples of the miRNA targeting p38 include miRNA containing, as a seed sequence, a nucleotide sequence complementary to a partial sequence (7 bases length) of the nucleotide sequence of mRNA encoding p38 (mature mRNA or primary transcript), and inhibiting transcription and/or translation of p38 can be mentioned.

miRNA representatively means what is called mature miRNA. The mature miRNA is an endogenous non-coding RNA having 20 - 25 bases, which is encoded on the genome. It is known that miRNA is transcribed as a primary transcript having a length of about several hundred - several thousand bases (Primary miRNA, hereinafter to be referred to as "Pri-miRNA") from the miRNA gene on the genomic DNA, then processed to be a pre-miRNA (precursor miRNA) having a hairpin structure of about 60 - 70 bases, after which moves from the nucleus into the cytoplasm, further processed to be a double stranded mature miRNA of about 20 - 25 bases, a single strand of the double stranded mature miRNA forms a complex with a protein called RISC, and acts on the mRNA of the target gene to inhibit translation of the target gene.

In the present specification, the term "miRNA" is used as a concept encompassing not only endogenous miRNA but also a synthetic nucleic acid consisting of the same nucleotide sequence as the endogenous miRNA.

The seed sequence means the nucleotide sequence of the 5'-terminal 2nd - 8th residues of the mature miRNA, and the seed sequence is known to determine the mRNA to be the target of miRNA.

miRNA targeting p38 can be obtained by searching, based on the nucleotide sequence of mRNA encoding p38 (for example, nucleotide sequence shown by SEQ ID NO: 1 or 3), for a miRNA having a seed sequence completely complementary to a partial sequence of the mRNA sequence (preferably, partial sequence of nucleotide sequence of 3'UTR of said mRNA), introducing the searched miRNA into the cell of p38 expressing cells, and selecting miRNA that suppressed expression of p38. The search of miRNA based on such target sequence can be performed based on target prediction programs such as TargetScan, PicTar, and miRanda.

Specifically, the miRNA targeting p38 is miR-17, miR-106a, and miR-106b. miR-17, miR-106a and miR-106b also include isomers of these miRNAs. The representative nucleotide sequences of miR-17, miR-106a and miR-106b are shown by SEQ ID NOs: 5, 6 and 7, respectively.

Also, a nucleic acid containing a nucleotide sequence having 85% or more, preferably 90% or more, more preferably 95% or more, identity with the nucleotide sequence shown by SEQ ID NO: 5, 6 or 7, and hybridizing, under physiological conditions, to an mRNA encoding p38, thereby inhibiting the transcription and/or translation thereof, is also encompassed in miRNA targeting p38.

The "identity" means a ratio (%) of identical nucleotide residues to all overlapping nucleotide residues in the optimal alignment where two nucleotide sequences are aligned using a mathematical algorithm known in the technical field (preferably, the algorithm considers introduction of gaps on one or both of the sequences for the best alignment).

In the present specification, nucleotide sequence identity can be calculated by, for example, aligning the two nucleotide sequences using the homology calculation algorithm NCBI BLAST-2 (National Center for Biotechnology Information Basic Local Alignment Search Tool) under the following conditions (gap open=5 penalties; gap extension=2 penalties; x_ dropoff=50; expectation value=10; filtering=ON).

The nucleotide sequence having 85% or more identity with the nucleotide sequence shown by SEQ ID NO: 5, 6 or 7 is a nucleotide sequence which is the nucleotide sequence shown by SEQ ID NO: 5, 6 or 7 wherein 1 to 3 nucleotides are deleted, substituted, inserted or added, for example, (1) a nucleotide sequence which is the nucleotide sequence shown by SEQ ID NO: 5, 6 or 7 wherein 1 - 3 (preferably 1 or 2) nucleotides are deleted, (2) a nucleotide sequence which is the nucleotide sequence shown by SEQ ID NO: 5, 6 or 7 wherein 1 - 3 (preferably 1 or 2) nucleotides are added, (3) a nucleotide sequence which is the nucleotide sequence shown by SEQ ID NO: 5, 6 or 7 wherein 1 - 3 (preferably 1 or 2) nucleotides are inserted, (4) a nucleotide sequence which is the nucleotide sequence shown by SEQ ID NO: 5, 6 or 7 wherein 1 - 3 (preferably 1 or 2) nucleotides are substituted by other nucleotides, or (5) a nucleotide sequence wherein the mutations of the above-mentioned (1) - (4) are combined (in this case, total of mutated nucleotides is 1 - 3, preferably 1 or 2).

In the nucleotide sequence having 85% or more identity with the nucleotide sequence shown by SEQ ID NO: 5, 6 or 7, a seed sequence common to miR-17, miR-106a and miR-106b is preferably maintained.

The precursor of miRNA means a nucleic acid that can produce mature miRNA in a cell as a result of intracellular processing or cleavage of double stranded nucleic acid. As the precursor, pri-miRNA, and pre-miRNA can be mentioned. The pri-miRNA is a primary transcript (single strand RNA) of miRNA gene, and generally has a length of about several hundred - several thousand bases. pre-miRNA is a single strand RNA having a hairpin structure, which results from intracellular processing of pri-miRNA, and generally has a length of 90 - 110 base. As pri-miRNA or pre-miRNA of miRNA, for example, the nucleotide sequence thereof is disclosed in miRBase database: http://micro RNA.sanger.ac.uk/ produced by Sanger Institute and the like.

The miRNA used in the present invention encompasses a nucleic acid at least partly containing RNA, which suppresses transcription and/or translation of p38 by RNA interference. Such nucleic acid is RNA, chimera nucleic acid of RNA and DNA (hereinafter to be referred to as chimera nucleic acid) or hybrid nucleic acid. As used herein, chimera nucleic acid refers to single strand or double strand nucleic acid containing RNA and DNA in a single nucleic acid, hybrid nucleic acid refers to double strand nucleic acid wherein one of the chains is RNA or chimera nucleic acid and the other chain is DNA or chimera nucleic acid.

The miRNA used in the present invention are single strand or double strand. The embodiments of double strand include double strand RNA, double strand chimera nucleic acid, RNA/DNA hybrid, RNA/chimera nucleic acid hybrid, chimera nucleic acid/chimera nucleic acid hybrid and chimera nucleic acid/DNA hybrid. The nucleic acid of the present invention is preferably single strand RNA, single strand chimera nucleic acid, double strand RNA, double strand chimera nucleic acid, RNA/DNA hybrid, RNA/chimera nucleic acid hybrid, chimera nucleic acid/chimera nucleic acid hybrid or chimera nucleic acid/DNA hybrid, more preferably single strand RNA, single strand chimera nucleic acid, double strand RNA, double strand chimera nucleic acid, RNA/DNA hybrid, chimera nucleic acid/chimera nucleic acid hybrid, RNA/chimera nucleic acid hybrid or an artificial nucleic acid constituted to include a molecule equivalent to natural nucleic acid.

The "antisense nucleic acid" refers to a nucleic acid comprising a nucleotide sequence capable of specifically-hybridizing with a target mRNA (mature mRNA or primary transcript) under physiological conditions for the cells that express the target mRNA, and capable of inhibiting the translation of the polypeptide encoded by the target mRNA in the hybridized state. The antisense nucleic acid may be a DNA or an RNA, and may be a DNA/RNA chimera, preferable DNA.

Examples of the antisense nucleic acid capable of specifically suppressing the expression of p38 include
(A) a nucleic acid comprising a nucleotide sequence complementary to the nucleotide sequence of an mRNA (mature mRNA or primary transcript product) that encodes p38 or a partial sequence thereof having a length of 12 bases or more; and
(B) a nucleic acid comprising a nucleotide sequence having a length of 12 bases or more which is capable of specifically hybridizing to an mRNA (mature mRNA or primary transcript) encoding p38, in the cell of an animal (preferably human) to be the subject of treatment, and capable of inhibiting, in the hybridized state, and translation into p38 polypeptide.

The length of the portion that hybridizes with the target mRNA in the antisense nucleic acid is not particularly limited as long as the expression of p38 is specifically suppressed. Generally, the length is about 12 bases or more, and as long as the full-length sequence of the mRNA (mature mRNA or primary transcript) for the longest. In view of hybridization specificity, the length is, for example, preferably about 15 bases or more, more preferably about 18 bases or more. In view of the ease of synthesis, antigenicity problems and the like, the length of the portion that hybridizes with the target mRNA is normally about 200 bases or less, preferably about 50 bases or less, more preferably about 30 bases or less. Hence, the length of the portion that hybridizes with the target mRNA is, for example, about 12 to about 200 bases, preferably about 15 to about 50 bases, more preferably about 18 to about 30 bases.

The target nucleotide sequence for the antisense nucleic acid is not particularly limited, as long as the expression of p38 can be specifically suppressed. The sequence may be the full-length sequence of an mRNA (mature mRNA or primary transcript) of p38 or a partial sequence thereof (e.g., about 12 bases or more, preferably about 15 bases or more, more preferably about 18 bases or more), or an intron portion of the primary transcript.

The nucleotide sequence of the portion that hybridizes with the target mRNA in the antisense nucleic acid varies depending on the base composition of the target sequence, and has an identity of normally about 90% or more (preferably 95% or more, most preferably 100%) to the complementary sequence for the target sequence, so as to be capable of hybridizing with the p38 mRNA under physiological conditions.

The size of the antisense nucleic acid is normally about 12 bases or more, preferably about 15 bases or more, more preferably about 18 bases or more. In view of the ease of synthesis, and antigenicity problems, the size is normally about 200 bases or less, preferably about 50 bases or less, more preferably about 30 bases or less.

Furthermore, the antisense nucleic acid may be one not only capable of hybridizing with the p38 mRNA or primary transcript thereof to inhibit the translation, but also capable of binding to the p38 gene, which is a double-stranded DNA, to form a triple strand (triplex) and inhibit the transcription into p38.

In natural nucleic acids, the phosphodiester bond is easily cleaved by a nuclease present in the cells. Therefore, the miRNA and an antisense nucleic acid used in the present invention may be modified to be a modified form resistant to the aforementioned nuclease. Examples of the modification to give a modified form include modified sugar chain moiety (e.g., 2'-O-methylation), modified base moiety, and modified phosphate or hydroxyl moieties (e.g., biotin, amino group, lower alkylamine group, acetyl group etc.).

siRNA and antisense nucleic acid targeting p38 can be prepared by determining the target sequence based on the mRNA sequence (e.g., the nucleotide sequence shown by SEQ ID NO:1 or 3) or chromosomal DNA sequence of p38, and synthesizing a nucleotide sequence complementary thereto using a commercially available automated DNA/RNA synthesizer (Applied Biosystems, Beckman and the like). An siRNA can be prepared by separately synthesizing a sense strand and antisense strand using an automated DNA/RNA synthesizer, denaturing the strands in an appropriate annealing buffer solution at about 90 to about 95°C for about 1 minute, and then annealing at about 30 to about 70°C for about 1 to about 8 hours. A longer double-stranded polynucleotide can be prepared by synthesizing complementary oligonucleotide strands in a way such that they overlap with each other, annealing the strands, and then performing ligation with a ligase.

The micro RNA targeting p38 can be obtained by searching, based on the mRNA sequence of p38 (for example, nucleotide sequence shown by SEQ ID NO: 1 or 3), for micro RNAs having a seed sequence complementary to a partial sequence of the mRNA sequence, preparing the searched micro RNAs by an automated DNA/RNA synthesizer (Applied Biosystems, Beckman and the like), introducing same into p38-expressing cells, and selecting a micro RNA actually suppressing the expression of p38.

The agent for promoting neuronal differentiation of the present invention can also contain, as an active ingredient, an expression vector capable of expressing (encoding) a nucleic acid which hybridizes, under physiological conditions, to a DNA or mRNA encoding p38, thereby inhibiting the transcription and/or translation thereof. In the expression vector, the nucleic acid which hybridizes, under physiological conditions, to a DNA or mRNA encoding p38, thereby inhibiting the transcription and/translation thereof, or a nucleic acid (preferably DNA) encoding same is operably linked to a promoter capable of exhibiting promoter activity in the cells (e.g., neural stem cell) of an mammal (preferably human or mouse) of application subject.

The promoter used is not particularly limited as long as it is functional in cells of the mammal of application subject. As the promoter, pol I promoter, pol II promoter, pol III promoter and the like can be used. Specifically, viral promoters such as the SV40-derived initial promoter and cytomegalovirus LTR, mammalian constitutive protein gene promoters such as the β-actin gene promoter, and RNA promoters such as the tRNA promoter, and the like are used.

When the expression of an siRNA or a micro RNA is intended, it is general that a pol III promoter be used as the promoter. As examples of the pol III promoter, the U6 promoter, H1 promoter, tRNA promoter and the like can be mentioned.

The above-mentioned expression vector preferably comprises a transcription termination signal, i.e., a terminator region, downstream of the above-described polynucleotide or nucleic acid that encodes the same. Furthermore, a selection marker gene for selection of transformant cells (a gene that confers resistance to a drug such as tetracycline, ampicillin, or kanamycin, a gene that compensates for an auxotrophic mutation and the like), and fluorescent protein genes (GFP, RFP, YFP etc.) can further be contained.

Although there is no limitation on the choice of expression vector used in the present invention, suitable vectors for administration to mammals such as humans include plasmid vector; viral vectors such as retrovirus, adenovirus, adeno-associated virus and lentivirus. Adenovirus, in particular, has advantages such as very high gene transfer efficiency and transferability to non-dividing cells as well. However, since the integration of transgenes into host chromosomes is extremely rare, the gene expression is transient and normally persists only for about 4 weeks. Considering the persistence of effect, it is also preferable to use adeno-associated virus and lentivirus, which offer a relatively high efficiency of gene transfer, which can be transferred into non-dividing cells as well, and which can be integrated into chromosomes.

Examples of the low molecular p38 inhibitor include, but not particularly limited to, pyridinylimidazoles, substituted pyrazoles, substituted pyridyls, quinazoline derivatives, aryl ureas, heteroaryl analogues, substituted imidazole compounds and substituted triazole compounds.

Many compounds have heretofore been proposed for p38 inhibition. The compounds proposed for p38 inhibition include pyridinylimidazoles. See Young P.R. et al. (1997) J. Biol. Chem. 272, pages 12116-12121; and Bender, P.E. (1985) J. Med. Chem. 28, pages 1169-1177. Examples of the pyridinylimidazoles capable of inhibiting p38 include 6-(4'-fluorophenyl)-5-(4'-pyridyl)-2,3-dihydroimidazo(2,1-b)-thiazole and a metabolite thereof (sulfoxide, sulfone), analogue thereof, fragment thereof and mimic thereof. Furthermore, it is proposed that 4-(pyridin-4-yl)-5-phenylimidazole, the smallest structure of the pyridinylimidazoles, could be sufficient to inhibit p38. See Gallagher, TF et al. (1997) Bio-org. Med. Chem. 5, pages 49-64.

Particular 1,5-diaryl substituted pyrazole compounds are also proposed as a p38 inhibitor. Such substituted pyrazole compounds are disclosed in US-B-6,509,361. Further pyrazole derivatives inhibiting p38 are disclosed in US-B-6,335,336.

Examples of other p38 inhibitors include substituted pyridyls such as those disclosed in US-B-2003/0139462.

Further p38 inhibitor are those disclosed in US-B-6,610,688.

A quinazoline derivative can also function as a p38 inhibitor. Examples of the quinazoline derivative as the p38 inhibitor are disclosed in US-B-6,541,477, 6,184,226, 6,509,363 and 6,635,644.

Aryl ureas and heteroaryl analogues can also function as p38 inhibitors. Examples of the aryl ureas and heteroaryl analogues as the p38 inhibitors are disclosed in US-B-6,344,476. WO 99/32110 describes hetero ring-ureas as the p38 inhibitor. WO 99/32463 describes urea compounds inhibiting p38 kinase. WO 98/52558 describes urea compounds for p38 kinase inhibition. WO 99/00357 describes a method for using urea compounds as a p38 kinase inhibitor. WO 99/58502 describes urea compounds as a p38 kinase inhibitor.

A substituted imidazole compound and a substituted triazole compound can also function as p38 inhibitors. Such compounds are respectively disclosed in US-B-6,560,871 and 6,599,910.

Examples of further p38 inhibitor include RWJ-67657 (RW Johnson Pharmaceutical Research Institute); RDP-58 (Sangstat Medical); RDP-58; Scios-469 (Scios); MKK3/MKK6 inhibitor (Signal Research Division); SB-210313 analogue, SB-220025, SB-238039, HEP-689, SB-203580, SB-239063, SB-239065, SB-242235 (SmithKline Beecham Pharmaceuticals); VX-702 and VX-745 (Vertex Pharmaceuticals); AMG-548 (Amgen); Astex p38 kinase inhibitor (Bayer); BIRB-796 (Boehringer Ingelheim Pharmaceuticals); Celltech p38 MAP kinase inhibitor (CeltechGroup plc.); FR-167653 (Fujisawa Pharmaceutical); 681323 and SB-281832 (GlaxoSmithKline plc); MAP kinase inhibitor of LEO Pharmaceuticals (LEO Pharma A/S); Merck&Co. p38 MAP kinase inhibitor (Merck research Laboratories); SC-040 and SC-XX906 (Monsanto); Novartis adenosine A3 antagonists (Novartis AG); p38 MAP kinase inhibitor (NovartisPharma AG); CP-64131 (Pfizer); CNI-1493 (Picower Institute for Medical Research); RPR-200765A (Phone-Poulenc Rorer); and Roche p38 MAP kinase inhibitor and Ro-320-1195 (Roche Bioscience).

In one embodiment, a low molecular p38 inhibitor is SB203580.

The agent for promoting neuronal differentiation of the present invention can contain, in addition to a p38 inhibitor, any carrier, for example, a pharmaceutically acceptable carrier.

Examples of the pharmaceutically acceptable carrier include excipients such as sucrose, starch, mannit, sorbit, lactose, glucose, cellulose, talc, calcium phosphate, calcium carbonate; binders such as cellulose, methylcellulose, hydroxypropylcellulose, polypropylpyrrolidone, gelatin, gum arabic, polyethylene glycol, sucrose, starch; disintegrants such as starch, carboxymethylcellulose, hydroxypropylstarch, sodium-glycol-starch, sodium hydrogen carbonate, calcium phosphate, calcium citrate; lubricants such as magnesium stearate, Aerosil, talc, sodium lauryl sulfate; aromatics such as citric acid, menthol, glycyrrhizin·ammonium salt, glycine, orange powder; preservatives such as sodium benzoate, sodium bisulfite, methylparaben, propylparaben; stabilizers such as citric acid, sodium citrate, acetic acid; suspensions such as methylcellulose, polyvinylpyrrolidone, aluminum stearate; dispersing agents such as surfactant; diluents such as water, saline, orange juice; base waxes such as cacao butter, polyethylene glycol, and white petroleum oil.

When a nucleic acid which hybridizes, under physiological conditions, to a DNA or mRNA encoding p38, thereby inhibiting the transcription and/or translation thereof, or an expression vector thereof is used as a p38 inhibitor, the agent for promoting neuronal differentiation of the present invention can further contain a reagent for nucleic acid introduction, to promote introduction of the nucleic acid into cells. As the reagent for nucleic acid introduction, a cationic lipid such as lipofectin, lipofectamine, lipofectamine RNAiMAX, Invivofectamine, DOGS (Transfectam), DOPE, DOTAP, DDAB, DHDEAB, HDEAB, polybrene, poly(ethyleneimine) (PEI) can be used. When retrovirus is used as an expression vector, RetroNectin, fibronectin, polybrene can be used as a reagent for introduction.

Examples of the dosage unit form of the agent of the present invention include liquid, tablet, pill, drinking liquid, powder, suspension, emulsion, granules, extract, fine granules, syrup, infusion, decoction, eye drop, troche, poultice, liniment, lotion, ophthalmic ointment, plaster, capsule, suppository, enema, injection (liquid, suspension and the like), adhesive preparation, ointment, jelly, paste, inhalant, cream, spray, nasal drop, and aerosol.

The content of the p38 inhibitor in the pharmaceutical composition is not particularly limited and is appropriately determined in a wide range. For example, it is 0.01 to 100 wt% of the whole pharmaceutical composition.

The agent of the present invention can be used for promoting neuronal differentiation of neural stem cells. In the present specification, the neural stem cell refers to a cell having an ability to differentiate into any type of cell constituting the central nervous system (neural multipotency), including neuron, astrocyte and oligodendrocyte, and having self replication potency. Neural stem cells generated from pluripotent stem cells such as fertilized eggs, embryonic stem cells, induced pluripotent stem cells and the like temporally changes their responsiveness to extrinsic differentiation-inducing stimulation while maintaining the multipotency. To be specific, in early neural stem cells, the neurogenic competence to differentiate into neurons in response to neurogenic signals is dominant, and the gliogenic competence to differentiate into astrocytes in response to gliogenic signals is recessive. However, along with the progress of differentiation stages, the neurogenic competence disappears, the gliogenic competence are acquired, and the cells transit into late neural stem cells wherein neurogenic competence is recessive and gliogenic competence is dominant. The present invention has been made based on the findings that p38 is involved in the transition of neural stem cells where neurogenic competence is dominant than gliogenic competence (neurogenic neural stem cell) into neural stem cells where gliogenic competence is dominant than neurogenic competence (gliogenic neural stem cell), specifically that p38 is involved in the acquisition of gliogenic competence of the neural stem cells, and expression of p38 is suppressed by micro RNA such as miR-17 in the early neural stem cells.

In the present specification, promotion of neuronal differentiation of neural stem cells means to increase neurogenic competence and decrease gliogenic competence of neural stem cells, or as a result of which enhance differentiation of neural stem cell into neuron. In one embodiment, the neural stem cells used in the present invention are gliogenic. When a p38 inhibitor is applied to the gliogenic neural stem cells, neurogenic competence increases and gliogenic competence is suppressed. As a result, conversion of gliogenic neural stem cells to neurogenic neural stem cells occurs. In one embodiment, the neural stem cells used in the present invention are neurogenic. When a p38 inhibitor is applied to the neurogenic neural stem cells, both a decrease in the neurogenic competence and acquisition of gliogenic competence are suppressed, and the neurogenic stage is maintained. In the present invention, both of these embodiments are encompassed in the promotion of neuronal differentiation of neural stem cells.

Whether the neural stem cells are neurogenic or gliogenic can be evaluated by culturing the evaluation target neural stem cells in the presence of glial differentiation inducing factors, LIF and BMP2, according to the method described in Nat. Neurosci. 11, 1014-1023 (2008), and examining which of the neurons and astrocytes are generated in a greater number. When the number of the astrocytes is higher than that of the neurons as a result of the culture, the neural stem cells are judged to be gliogenic; otherwise, the neural stem cells can be judged to be neurogenic.

In the present invention, the neural stem cells may be present in vivo or in vitro. Therefore, the agent for promoting neuronal differentiation of the present invention can be used as an in vivo medicament or a reagent for experiment in vitro.

Since the neural stem cells are present in vivo in the cerebral cortex in the embryonic stage/fetal stage, and hippocampus and lateral cerebral ventricle of adult, in human or non-human mammal (e.g., mouse), a p38 inhibitor (or the agent for promoting neuronal differentiation of the present invention) is administered to an individual mammal such that it is delivered to the neural stem cells in the neural tissues in the body, whereby neuronal differentiation of neural stem cells can be promoted in vivo.

The disease to be treated is a disorder selected from cerebral infarction, traumatic brain injury, cerebral ischemia, Huntington's disease, and Alzheimer's disease. like.

When in use, the agent for promoting neuronal differentiation of the present invention is administered according to a method suitable for each form. For example, in the case of an external preparation, the agent is sprayed, adhered or applied directly onto the required site such as skin or mucosa; in the case of tablet, pill, drinking liquid, suspension, emulsion, granule or capsule, the agent is administered orally; in the case of injection, the agent is administered intravenously, intracerebrally, intramuscularly, intradermally, subcutaneously, intraarticularly or intraperitoneally; and in the case of suppository, the agent is administered intrarectally.

While the dose of the agent of the present invention varies depending on the activity and choice of the active ingredient, administration manner (e.g., oral, parenteral), recipient animal species, the recipient's drug receptivity, body weight, age, and the like, and cannot be generalized, the dose is normally about 0.001 mg to about 2.0 g, based on the amount of active ingredient, per day for an adult.

Moreover, when the agent for promoting neuronal differentiation of the present invention is used in vitro, the neuronal differentiation of the neural stem cells can be promoted by cultivating the neural stem cells in the presence of a p38 inhibitor (or the agent for promoting neuronal differentiation of the present invention). The present invention also provides an in vitro method of promoting neuronal differentiation of neural stem cells.

While the neural stem cells in a culture state may be obtained by isolating neural stem cells in the living body and culturing same, they may also be prepared by differentiating pluripotent stem cells such as embryonic stem cells (ES cell), induced pluripotent stem cells (iPS cell) and the like into the neural stem cells. A method of inducing differentiation of ES cells into neural stem cells is disclosed in detail in JP-A-2002-291469. The method is also applicable to other pluripotent stem cells such as iPS cell.

To enhance differentiation potency of pluripotent stem cells into the nerve system in the differentiation induction into neural stem cells, it is preferable to form embryoid (embryoid body; EB) in advance and differentiate the EB into neural stem cells under particular culture conditions. EB can be formed by cultivating ES cells or iPS cells in the presence of, for example, retinoic acid or Noggin protein. In the case of retinoic acid, it may be added to a medium at a low concentration level (10⁻⁹ M - 10⁻⁶ M). In the case of Noggin protein, a culture supernatant obtain by introducing Xenopus Noggin into mammalian cultured cells, and allowing for transient expression of the Noggin protein may be directly (1 - 50%(v/v)) used, or a recombinant Noggin protein (final concentration about 1 µg/ml) may also be used.

The thus-obtained EB is dissociated and subjected to suspension culture in a serum-free medium added with FGF-2 (generally 10 - 100 ng/ml), whereby neural stem cells can be obtained in the form of neurosphere. While the serum-free medium is not particularly limited as long as it is a serum-free medium generally used for culturing neural stem cells or neurons, examples thereof include a DMEM medium added with, besides the above-mentioned components, glucose, glutamine, insulin, transferrin, progesterone, putrescine, selenium chloride, and heparin. The medium to be used for neurosphere formation may be added with a sonic hedgehog protein (generally, 1 - 20 nM). Since addition of a sonic hedgehog protein improves differentiation induction efficiency of neural stem cells into neural progenitor cells and growth efficiency thereof, the differentiation efficiency into neurons by differentiation culture thereafter is enhanced. The culture is preferably performed under 5% CO₂ conditions at 35 - 40°C for about 7 - 9 days.

In this case, a neurosphere directly derived from EB is referred to as a primary neurosphere, and a neurosphere reformed by separating and dispersing the primary neurosphere and cultivating same under the same conditions is referred to as a secondary neurosphere. In the following, a neurosphere passaged even only once under culture conditions is referred to as a secondary neurosphere. Generally, neural stem cells contained in the primary neurosphere are neurogenic. Neural stem cells contained in the secondary neurosphere generally have lower neurogenic competence and higher gliogenic competence as compared to the neural stem cells contained in the primary neurosphere. In one embodiment, neural stem cells contained in the secondary neurosphere are gliogenic.

When the thus-obtained neurosphere containing neural stem cells is cultivated under nerve cell differentiation conditions, differentiation of the neural stem cells into neurons is induced. The neuronal differentiation is promoted by the addition of a p38 inhibitor (or the agent for promoting neuronal differentiation of the present invention) in the neurosphere stage. When a nucleic acid which hybridizes, under physiological conditions, to a DNA or mRNA encoding p38, thereby inhibiting the transcription and/or translation thereof, or an expression vector thereof is used as a p38 inhibitor, a reagent for nucleic acid introduction may also be used to promote introduction of the nucleic acid into cells. While the neuronal differentiation conditions are not particularly limited, for example, culture in a medium free of mitogen such as FGF-2 can be mentioned. As the medium, conventional media applicable for culturing neural stem cells and neurons can be used as long as mitogen is not contained. Examples thereof include DMEM:F12 medium containing glucose, glutamine, insulin, transferrin, progesterone, putrescine, and selenium chloride. Sonic hedgehog protein may be present or absent. Examples of other neuronal differentiation conditions include those described in Nat Biotechnol. 21, 183-186 (2003) and those of ordinary skill in the art can easily construct various neuronal differentiation conditions. A differentiation culture into neuron is preferably performed under 5% CO₂ conditions at 35 - 40°C for 5 - 7 days. When a primary neurosphere is used as a neurosphere, the p38 inhibitor acts on neurogenic neural stem cells contained therein, and both a decrease in the neurogenic competence and acquisition of the gliogenic competence are suppressed and the neurogenic stage of the neural stem cells is maintained. As a result, differentiation of neural stem cells into neurons is promoted. When a secondary neurosphere is used as a neurosphere, the p38 inhibitor acts on neural stem cells having the gliogenic competence (preferably gliogenic neural stem cells) contained therein to increase the neurogenic competence and suppress the gliogenic competence. As a result, restoration of differentiation potency is occurred into a neurogenic neural stem cell and differentiation of neural stem cells into neurons is promoted.

The present invention is explained in more detail in the following by referring to the Examples shown below.

### Examples

### <Material and method>

### Cell culture and neurosphere differentiation assay

Mouse ESC (EB3) culture, EB formation, neurosphere formation and differentiation of neurosphere were performed as previously described (Nat. Neurosci. 11, 1014-1023 (2008)). Human NSCs were grown as previously described (J. Neurosci. Res. 69, 869-879 (2002); J. Neurosci. Res. 87, 307-317 (2009)) without epithelial growth factor (EGF), and allowed to differentiate on a substrate-coated coverslips in the absence of mitogen for 14 days, a procedure similar to that used for mouse neurosphere.

### Lentivirus preparation

Lentivirus particles were produced by the transient transfection of 293T human embryonic kidney cells with a lentiviral expression construct for the gene of interest, along with two lentivirus constructs required for viral replication (pCMV-VSV-G-RSV-Rev and pCAG-HIVgp). The transfection was performed using GeneJuice (Novagen) according to the manufacturer's instructions. The virus particles were collected by ultracentrifugation and then resuspended in phosphate-buffered saline. High-titer viral suspensions were used to obtain an efficient infection of NSCs with a multiplicity of infection of about 5.

### Animal and uterus virus infection

Experiments were performed using ICR mice. All aspects of animal care and treatment were carried according to the guidelines of the Experimental Animal Committee of Keio University School of Medicine. In utero microinjection of lentivirus particles into the brain of E10.5 ICR mouse was guided by an in vivo ultrasound real-time scanner (Vevo660; VisualSonics).

### Immunostaining

Immunocytechemistry and immunohistochemistry were performed as previously described (Nat. Neurosci. 11, 1014-1023 (2008)) using antibodies against βIII-tubulin, NeuN (neural marker), glial fibrillary acidic protein (GFAP, astrocyte marker), and haemagglutinin (HA).

### Proteomics analysis

Quantitative proteomics analyses using an iTRAQ method were performed for miR-LacZ-, and miR-17s-overexpressing p0 and p2 neurospheres, using AB SCIEX TripleTOF 5600 System (AB SCIEX) according to the manufacturer's instructions.

### Statistical analysis

At least three independent experiments were performed for each statistical analysis. The statistical significance was determined by two-tailed t-test. Throughout the study, P<0.05 was considered to be significantly different.

### <Results>

### Identification of genes acting downstream of Coup-tfs

The present inventors began this experiment by identifying candidate genes at the downstream of Coup-tfs. The direct DNA-binding sites of Coup-tfs were investigated using chromatin immunoprecipitation sequencing (ChIP-seq), and global gene and microRNA expression profiles were compared between control and Coup-tfs knockdown mouse neurospheres by microarray analysis (Nat. Neurosci. 11, 1014-1023 (2008)). Based on the obtained data, candidate genes were acquired and lentiviral libraries were constructed for their overexpression and knockdown. Next, the present inventors used the neurosphere culture system to functionally screen the candidate genes as previously described (Nat. Neurosci. 11, 1014-1023 (2008)). Finally, the miR-17-92 microRNA gene cluster was identified as a downstream effector of Coup-tfs.

The miR-17-92 cluster was first identified as a cluster of oncogenic microRNAs, namely OncomiR-1, and was associated with the development of various organs (Nature 435, 828-833 (2005); Cell 133, 217-222 (2008); Cell 132, 875-886 (2008)). According to microarray analysis, the expression of this gene cluster was 3.58-fold higher in twice-passaged (p2) Coup-tfs knockdown neurospheres than in control neurospheres. Normally, the majority of cells in p2 neurospheres differentiate into astrocytes, with less than 20% becoming neurons. However, overexpression of the miR-17-92 cluster caused them to differentiate almost exclusively into neurons, with the elaboration of long βIII-tubulin-positive processes (Fig. 1).

The miR-17-92 cluster encodes six distinct microRNAs (miR-17, 18a, 19a, 20a, 19b and 92a, with their star (*) strands). To determine which of the six microRNAs was responsible for the neuronal phenotype, the present inventors individually overexpressed each of them in NSCs. This was done by infecting NSCs with lentiviruses expressing microRNAs and green fluorescent protein (GFP). Of the six microRNAs, only miR-17 replicated the phenotype induced by the intact miR-17-92 cluster (Fig. 1). The nucleotide sequences of miR-17 and miR-20a are highly homologous and share the same 7-mer seed sequence (nucleotides 2-8, Fig. 2) that is required for binding to target mRNAs. Therefore, the target mRNAs of these two microRNAs are also speculated to have highly overlapping sequences (Cancer Res. 68, 8191-8194 (2008)). However, the overexpression of miR-20a did not yield a neuronal promoting action (Fig. 1).

MiR-17 has two additional paralogs with high homology and identical seed sequences, miR-106b and miR-106a, which are encoded by the miR-106b-25 and miR-106a-363 clusters, respectively (Cancer Res. 68, 8191-8194 (2008)). These paralogs were individually overexpressed in NSCs to examine whether either or both affected the neurogenesis-to-gliogenesis switch. Overexpression of both microRNAs led to a neuronal phenotype, although the effect of miR-106b was stronger than that of miR-106a (Fig. 1). Thus, miR-17, miR-106a and miR-106b were collectively termed "miR-17s" in Examples of the description.

As demonstrated previously, knockdown of Coup-tfs causes sustained generation of Islet-1 (Isl-1)-expressing neurons, a subpopulation of early-born neurons found even at the p2 neurosphere stage (Nat. Neurosci. 11, 1014-1023 (2008)). Notably, overexpression of miR-17s did not result in the cytogenesis of Isl-1-positive neurons, even though miR-17s were identified as downstream effectors of Coup-tfs. Instead, Onecut2 was identified as a positive regulator of Isl-1-positive cell production during candidate gene screening. These results suggested that both neurogenesis-to-gliogenesis transition in NSCs and neuron subtype specification are independently regulated downstream of Coup-tfs.

The transition from early-developmental neurogenic NSCs to late-developmental gliogenic NSCs can be recognized by changes in the responsiveness of NSCs to gliogenic cytokines (Nat. Neurosci. 11, 1014-1023 (2008)). LIF and BMP2 are well-known extrinsic glial differentiation inducing factors that promote gliogenesis only in late-developmental NSCs by activating the JAK-STAT pathway and BMP signaling, respectively (Proc. Natl. Acad. Sci. U. S. A. 95, 3178-3181 (1998); Science 284, 479-482 (1999); Proc. Natl. Acad. Sci. U. S. A. 98, 5868-5873 (2001)). LIF does not act as a gliogenic factor in early-developmental NSCs because the STAT3-binding site in the promoter of glial marker genes is epigenetically suppressed (epigenetically silenced) by DNA methylation (Dev. Cell 1, 749-758 (2001)). BMP signaling, for its part, promotes neuronal rather than glial differentiation in early-developmental NSCs (Proc. Natl. Acad. Sci. U. S. A. 98, 5868-5873 (2001); J. Neurosci. 18, 8853-8862 (1998)). Therefore, to examine whether miR-17 alters the responsiveness of NSCs to glial differentiation induction cytokines, miR-17-overexpressing NSCs were exposed to LIF and BMP2 during differentiation. MiR-17-overexpressing neurospheres were strongly resistant to these cytokines and differentiated exclusively into neurons, even at the p2 stage (Fig. 3).

The present inventors next investigated the temporally-regulated DNA methylation status of the STAT3-binding site and its surrounding CpG sites in the Gfap promoter. Gfap is the most general gene marker for astrocytes. However, no significant changes in the extent of CpG methylation at the p2 neurosphere stage were observed following the overexpression of miR-17 (Fig. 4). Coup-tfs are transiently upregulated in developing NSCs during mid-gestation. After this time, NSCs lose their plasticity and produce only late-born subtype neurons and glial cells. Because knockdown of Coup-tfs in ESC-derived neurospheres results in the maintenance of the early epigenetic status of the Gfap promoter (Nat. Neurosci. 11, 1014-1023 (2008)), the present inventors initially expected that the expression of the molecular entities (e.g., miR-17s) responsible for the changes in NSC competence would converge with changes in the epigenetic status of neuronal or glial differentiation-associated genes. However, the current results suggest that miR-17s function at another stage in Coup-tfs-regulated competency transition. The change in the epigenetic status of cell-type specific genes may therefore be only one necessary prerequisite for the control of cytogenesis.

### MiR-17s as determinants for the neurogenic competence of NSCs

To ensure that miR-17s are downstream effectors of Coup-tfs, the present inventors next performed experiments using RNA decoys, namely "tough decoy (TuD)" RNA, which strongly and stably suppresses specific target microRNAs (Nucleic Acids Res 37, e43 (2009)). Lentivirus vectors were constructed that expressed miR-17-specific TuD, miR-17*-specific TuD (TuD-miR-17 and TuD-miR-17*, respectively), or control TuD-miR-LacZ.

This series of experiments employed neurospheres in which Coup-tfs was silenced using Coup-tfs-specific short hairpin RNA (shRNA) from the p0 stage (Nat. Neurosci. 11, 1014-1023 (2008)). The neurogenesis-to-gliogenesis switch is inhibited in these suppressed (silenced) neurospheres, as it is in miR-17-overexpressing neurospheres. As expected, TuD-miR-17 expression largely cancelled out the neuronal differentiation promoting action induced by Coup-tfs knockdown at the p2 stage (Fig. 5), whereas the TuD-miR-17* and Tud-mi-17-LacZ controls had no significant effect. Moreover, while p0 neurospheres barely differentiate into glia, even in the presence of gliogenic cytokines, the forced expression of TuD-miR-17 (but not TuD-miR-17*) resulted in the induction of precocious gliogenesis, but only in the presence of LIF and BMP2 (Fig. 6). These results clearly demonstrate that miR-17s are responsible for the neurogenic competence of NSCs, at least in part due to their actions downstream of Coup-tfs.

### Functions of miR-17s in the developing CNS

As a first step toward exploring the functional roles of miR-17s in vivo, the present inventors investigated their expression patterns in the developing CNS. According to the microarray data, expression levels of miR-17 and miR-106b were 0.18- and 0.28-fold lower in p2 neurospheres than in p0 neurospheres. As expected from these data, the in vivo expression patterns of miR-17 and miR-106b were similar at embryonic Day 11.5 (E11.5) and postnatal Day 0 (P0), although a stronger signal was observed for the former at E11.5. The levels of both microRNAs declined as development progressed (Fig. 7). Next, the in vivo roles of miR-17s were analyzed by examining the effects of miR-17 overexpression in the developing mouse brain via in utero lentiviral microinjection. Consistent with the in vitro results, most of the cells (97.6%) infected with the miR-17-overexpressing lentivirus at E10.5 were fated to become neurons in the cerebral cortex by P30, whereas only 62.2% of the cells infected with the control miR-LacZ-expressing lentivirus became neurons (Fig. 8). Thus, overexpression of miR-17 also inhibited gliogenesis in vivo in a cell-autonomous fashion.

### Identification of p38 as a direct target for miR-17s

Normally, a microRNA regulates the degradation and/or translation of multiple target mRNAs (Biogerontology 11, 501-506 (2010)). To further identify the molecular mechanisms underlying changes in the competence of NSCs, the present inventors tried to identify the target mRNAs of miR-17s. To this end, the present inventors first carried out a proteomics analysis using the iTRAQ (isobaric tags for relative and absolute quantitation) method to identify proteins downregulated by the overexpression of miR-17 in p2 neurospheres (17). Candidate genes were then scanned using our original "in-house" computer program "HIMAJA", which searches for sequences in mRNAs that are complementary to the seed sequences of particular microRNAs. An initial list of 40 genes was obtained. Finally, mRNA encoding p38 (also known as mitogen-activated protein kinase 14, or Mapk14) was identified as a direct target for miR-17s during the regulation of competence transition of NSCs as follows.

First, neurospheres at the p2 stage showed higher neuropotency than controls following exposure to a specific p38 inhibitor (SB203580) (Fig. 9), as did p2 neurospheres following knockdown of p38 using a p38-specific shRNA (Fig. 10). On the other hand, p38 protein expression increased during the passage of neurospheres (3.16-fold higher at p2 than at p0). In the Western blotting, the expression is 2.78-fold higher at p2 than at p0 (Fig. 18). Next, a reporter assay using the miR-17s-binding site in the 3'untranslated region (UTR) of p38 mRNA revealed a direct interaction between p38 mRNA and miR-17 (Fig. 17). Finally, the overexpression of miR-17s-resistant p38 mRNA, which lacks the entire 3'UTR, not only reversed the neuronal phenotype induced by miR-17 overexpression to a glial phenotype (Fig. 11) but also induced precocious gliogenesis from p0 neurospheres (Fig. 12). These results indicate that p38 is a direct target for miR-17s and is largely involved in the initiation of gliogenesis.

### Forced restoration of neuropotency in gliogenic NSCs

Finally, the present inventors asked whether the overexpression of miR-17s could restore neuropotency in stage-progressed NSCs. To address this, the present inventors overexpressed miR-17 in neurospheres via lentiviral infection at the onset of the p3 stage, when neurospheres normally differentiate exclusively into glial cells. Previous work of the present inventors showed that knockdown of Coup-tfs resulted in the recovery of neuropotency only in limited populations of p3 NSCs, which had apparently not yet lost their plasticity (Nat. Neurosci. 11, 1014-1023 (2008)). Surprisingly, however, overexpression of miR-17 led to a marked recovery of neuropotency in p3 neurospheres within 1 week, whereas the vast majority of cells within control p3 neurospheres differentiated into astrocytes (Fig. 13). In addition, the DNA methylation status of the Gfap promoter did not change significantly (Fig. 14), as observed previously during continuous upregulation of miR-17 in neurospheres from the p0 stage (Fig. 4).

The specific p38 inhibitor, SB203580, and overexpression of p38 restored neuropotency (Fig. 15) and promoted gliogenesis (Fig. 16), respectively, in human fetal brain-derived neurospheres (J. Neurosci. Res. 69, 869-879 (2002); J. Neurosci. Res. 87, 307-317 (2009)). These results suggest that the molecular machinery directing the neuronal-to-glial competence transition of NSCs is evolutionarily conserved, but that its microRNA-mediated regulation differs among species.

### Functional roll of p38 in vivo in developing neural stem/progenitor cells (NSPCs)

To determine the in vivo roles of p38, gain- and loss-of-function studies were performed by in utero lentiviral microinjection. Consistent with the in vitro results, when p38 was overexpressed, precocious GFAP protein expression was observed in the cerebral cortex at E17.5. In utero injections of p38-overexpressing lentivirus resulted in the generation of cell clusters with low-level GFAP protein expression in the subventricular zone (SVZ). In contrast, control cells overexpressing eGFP did not form cell clusters or express GFAP (Fig. 19). p38 knockdown experiments using a lentivirus expressing p38-specific shRNA revealed a decrease in the numbers of VZ and SVZ cells expressing acyl-CoA synthase bubblegum family 1 (Acsbg1) at E17.5 (Fig. 20). Acsbg1 is a gray matter astrocyte marker that is useful for the detection of GFAP-positive mature astrocytes and GFAP-negative immature astrocytes in the cerebral cortex (J Neurosci 28, 264-278 (2008)). These observations suggest that p38 is critically involved in the initiation of gliogenesis. Taken together, the above-mentioned results teach that the miR-17/106-p38 axis is a major regulator of the neurogenic-to-gliogenic transition in developing neural stem/progenitor cells, and that manipulation of this axis permits bidirectional control of the multipotency of neural stem/progenitor cells.

### Industrial Applicability

According to the present invention, differentiation of neural stem cells into neurons can be promoted by new mechanism of p38 suppression. This is based on the restoration of the neurogenic competence in gliogenic neural stem cells, and neuronal differentiation can be exclusively induced.

This application is based on a patent application No. 2012-103875 filed in Japan (filing date: April 27, 2012).

## Claims

1. A p38 inhibitor selected from miR-17, miR-106a and miR-106b for use in a method of treating or preventing a disorder selected from cerebral infarction, traumatic brain injury, cerebral ischemia, Huntington's disease and Alzheimer's disease.

2. An agent comprising the p38 inhibitor according to claim 1 and a pharmaceutically acceptable carrier for use according to claim 1.

3. An *in vitro* method of promoting neuronal differentiation of neural stem cells, comprising cultivating neural stem cells in the presence of the p38 inhibitor as defined in claim 1.

4. The method according to claim 3, wherein the neural stem cells are gliogenic.

5. The method according to claim 3, wherein the neural stem cells are neurogenic.

6. An *in vitro* method of converting a gliogenic neural stem cell to a neurogenic neural stem cell, comprising cultivating gliogenic neural stem cells in the presence of the p38 inhibitor as defined in claim 1.

## Patentansprüche

1. p38-Inhibitor, ausgewählt aus miR-17, miR-106a und miR-106b zur Verwendung in einem Verfahren zur Behandlung oder Prävention einer Störung, die aus Hirninfarkt, Schädel-Hirn-Trauma, zerebraler Ischämie, Chorea Huntington und Alzheimer-Krankheit ausgewählt ist.

2. Mittel, das den p38-Inhibitor gemäß Anspruch 1 und einen pharmazeutisch annehmbaren Träger zur Verwendung gemäß Anspruch 1 umfasst.

3. In-vitro-Verfahren zur Förderung der neuronalen Differenzierung neuraler Stammzellen, umfassend das Kultivieren neuraler Stammzellen in Gegenwart des p38-Inhibitors gemäß Anspruch 1.

4. Verfahren gemäß Anspruch 3, wobei die neuralen Stammzellen gliogen sind.

5. Verfahren gemäß Anspruch 3, wobei die neuralen Stammzellen neurogen sind.

6. In-vitro-Verfahren zur Umwandlung einer gliogenen neuralen Stammzelle in eine neurogene neurale Stammzelle, umfassend das Kultivieren gliogener neuraler Stammzellen in Gegenwart des p38-Inhibitors gemäß Anspruch 1.

## Revendications

1. Inhibiteur de p38 sélectionné parmi miR-17, miR-106a et miR-106b pour son utilisation dans une méthode de traitement ou de prévention d'un trouble sélectionné parmi l'infarctus cérébral, le traumatisme crânien, l'ischémie cérébrale, la maladie de Huntington et la maladie d'Alzheimer.

2. Agent comprenant l'inhibiteur de p38 selon la revendication 1 et un support pharmaceutiquement acceptable pour son utilisation selon la revendication 1.

3. Méthode *in vitro* pour favoriser la différenciation neuronale de cellules souches neurales, comprenant la culture des cellules souches neurales en présence de l'inhibiteur de p38 tel que défini dans la revendication 1.

4. Méthode selon la revendication 3, dans laquelle les cellules souches neurales sont gliogéniques.

5. Méthode selon la revendication 3, dans laquelle les cellules souches neurales sont neurogéniques.

6. Méthode *in vitro* pour convertir une cellule souche neurale gliogénique en une cellule souche neurale neurogénique, comprenant la culture des cellules souches neurales gliogéniques en présence de l'inhibiteur de p38 tel que défini dans la revendication 1.
